(19) Europäisches Patentamt
European Patent Office
Office européen des brevets

(11) EP 4 700 050 A1

(12) EUROPEAN PATENT APPLICATION
published in accordance with Art. 153(4) EPC

(43) Date of publication:
25.02.2026 Bulletin 2026/09

(21) Application number: 24797048.6

(22) Date of filing: 24.04.2024

(51) International Patent Classification (IPC):
C08F 22/14 (2006.01) C08J 5/18 (2006.01)
G09F 9/00 (2006.01)

(52) Cooperative Patent Classification (CPC):
C07C 69/60; C08F 22/14; C08J 5/18;
C07C 2601/14; G09F 9/00

(86) International application number:
PCT/JP2024/016031

(87) International publication number:
WO 2024/225302 (31.10.2024 Gazette 2024/44)

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
GE KH MA MD TN

(30) Priority: 27.04.2023 JP 2023073591

(71) Applicants:
- Tosoh Corporation
  Yamaguchi 746-8501 (JP)
- University Public Corporation Osaka
  Osaka-shi, Osaka 5360025 (JP)

(72) Inventors:
- WATANABE Misaki
  Yokkaichi-Shi, Mie 510-8540 (JP)
- MURAKAMI Noritake
  Yokkaichi-Shi, Mie 510-8540 (JP)
- YOSHIMURA Asuka
  Yokkaichi-Shi, Mie 510-8540 (JP)
- KOMINE Takuya
  Yokkaichi-Shi, Mie 510-8540 (JP)
- KITAGAWA Takahiro
  Yokkaichi-Shi, Mie 510-8540 (JP)
- MATSUMOTO Akikazu
  Sakai-shi, Osaka 599-8531 (JP)
- SUZUKI Yasuhito
  Sakai-shi, Osaka 599-8531 (JP)

(74) Representative: Novagraaf International SA
Chemin de l'Echo 3
1213 Onex, Geneva (CH)

# (54) NOVEL FUMARIC ACID ESTER MONOMER, RESIN, AND FILM CONTAINING SAME

(57) Provided are: a novel fumaric acid ester monomer that yields a resin having high $T_\beta$; a resin; and a film containing the same.

This fumaric acid ester monomer is represented by formula (1). In the formula, $R_1$ represents a linear alkyl group having 1 to 4 carbon atoms or a branched alkyl group having 3 to 5 carbon atoms, and $R_2$ represents a linear alkyl group having 1 to 4 carbon atoms, a branched alkyl group having 3 to 12 carbon atoms, or a cyclic alkyl group having 3 to 6 carbon atoms.

EP 4 700 050 A1

[Chem. 1]

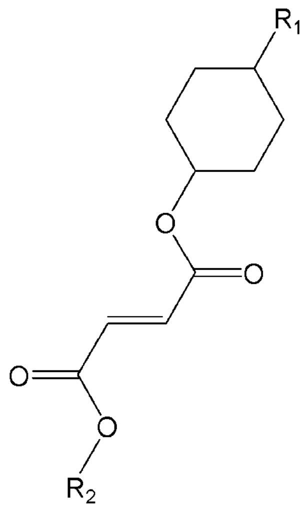

(1)

## Description

TECHNICAL FIELD

The present disclosures relate to a novel fumaric acid ester monomer, a resin, and a film containing the same.

BACKGROUND ART

Liquid crystal displays are the most important displays in the multimedia community, and are used so widely as in mobile phones, computer monitors, notebook personal computers and televisions. In the polarizing plate used in liquid crystal displays, many optical films are being used for contrast improvement when viewed from the front or an angle, and display characteristic improvements such as color tone compensation.

As a representative film in the polarizing plate-related optical films, a retardation film can be exemplified. The retardation film can be used as an antireflection layer of a liquid crystal display device, a touch panel or organic EL.

As a conventional retardation film, polycarbonates or cyclic polyolefins can be used, and these polymers are all polymers having positive birefringence. Herein, the positive and negative of birefringence are defined as indicated below.

The optical anisotropy of the polymer film molecularly oriented by stretching, casting by a coater or the like can be represented by the indicatrix defining the refractive index in the fast axis direction (direction of smallest refractive index) in the film plane as nx, and the refractive index in a film in-plane direction (slow axis) orthogonal to this as ny, and the refractive index in the thickness direction of the film (perpendicular direction outside of the film plane) as nz.

In other words, the refractive index in the stretching axis direction is small in uniaxial stretching of polymers having negative birefringence (fast axis: stretching direction), and the refractive index in the axial direction orthogonal to the stretching axis direction in uniaxial stretching of a polymer having positive birefringence is small (fast axis: orthogonal direction to stretching direction).

Many polymers have positive birefringence. As a polymer having negative birefringence, there are acrylic resins and polystyrenes; however, acrylic resins have little retardation developability, and thus the characteristics as a retardation film are not sufficient. Polystyrenes have problems in terms of optical characteristics such as a large wavelength dependence of retardation, in terms of practical use such as low heat resistance, and a problem of retardation stability such as the photoelasticity being large in the room temperature region and the retardation changing with even slight stress, and thus are not currently being used.

There is strong market demand for retardation films exhibiting such negative birefringence. Various retardation films have been developed to address the above demanded characteristics.

As optical films having high refractive index in the thickness direction exhibiting negative birefringence, a fumaric acid ester resin have been proposed in Patent Documents 1 and 2.

Citation List

Patent Document

Patent Document 1: Japanese Unexamined Patent Application, Publication No. 2008-064817
Patent Document 2: Japanese Unexamined Patent Application, Publication No. 2011-107281

DISCLOSURE OF THE INVENTION

Problems to be Solved by the Invention

In recent years, accompanying the diversification of display usage environments, a retardation film has been demanded with high heat resistance such that the dimensions thereof hardly change even if used in a high-temperature environment. Although reporting on fumaric acid ester resins has been made in Patent Documents 1 and 2, fumaric acid ester resins having higher heat resistance has been demanded.

Fumaric acid ester resins have a β relaxation temperature ($T_\beta$) corresponding to the side chain movement of the resin, and when the $T_\beta$ is within the display usage environment temperature range or lower, the dimensional stability of the film containing this resin becomes low. For this reason, in order to further raise the heat resistance, a fumaric acid ester resin having high $T_\beta$ is necessary.

The present invention has been made taking the above situation into account, and has an object of providing a novel fumaric acid ester monomer that yields a resin having high $T_\beta$, a resin, and a film containing the same.

Means for Solving the Problems

As a result of the thorough investigation by the present inventors, it was found that the above-mentioned problems could be solved by a specific fumaric acid ester monomer, thereby arriving at completion of the present invention.

That is, the present disclosure has the following key points.

[1] A fumaric acid ester monomer is represented by formula (1) below.

[Chem. 1]

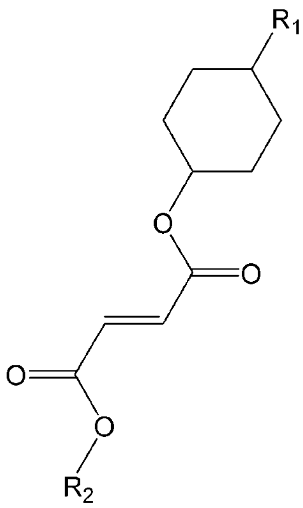

(1)

(In the formula, $R_1$ represents a linear alkyl group having 1 to 4 carbon atoms or a branched alkyl group having 3 to 5 carbon atoms. $R_2$ represents a linear alkyl group having 1 to 4 carbon atoms, a branched alkyl group having 3 to 12 carbon atoms or a cyclic alkyl group having 3 to 6 carbon atoms.)

[2] In the fumaric acid ester monomer as described in [1], $R_1$ is a methyl group, ethyl group, propyl group, isopropyl group, butyl group, tert-butyl group, isobutyl group, sec-butyl group, sec-pentyl group or tert-pentyl group.

[3] In the fumaric acid ester monomer as described in [1] or [2], $R_2$ is a methyl group, ethyl group, propyl group, isopropyl group, butyl group, isobutyl group, sec-butyl group, sec-pentyl group, tert-pentyl group, sec-hexyl group, tert-hexyl group, cyclopropyl group, cyclopentyl group or cyclohexyl group.

[4] The fumaric acid ester monomer as described in any one of [1] to [3] has a melting point of 80°C or lower.

[5] A resin includes a residue unit represented by structural formula (2) below.

[Chem. 2]

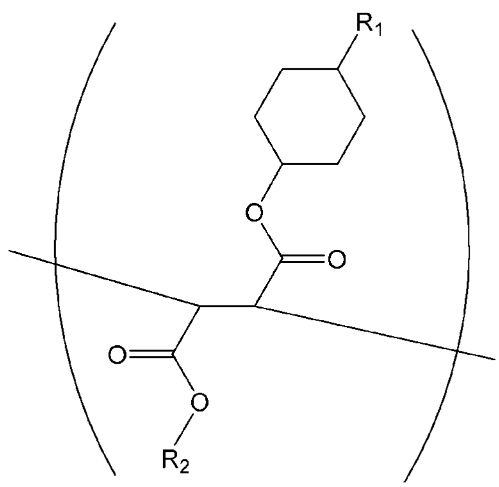

(2)

(In the formula, $R_1$ represents a linear alkyl group having 1 to 4 carbon atoms or a branched alkyl group having 3 to 5 carbon atoms. $R_2$ represents a linear alkyl group having 1 to 4 carbon atoms, a branched alkyl group having 3 to 12 carbon atoms or a cyclic alkyl group having 3 to 6 carbon atoms.)

[6] The resin as described in [5] further includes a residue unit represented by structural formula (3) below.

[Chem. 3]

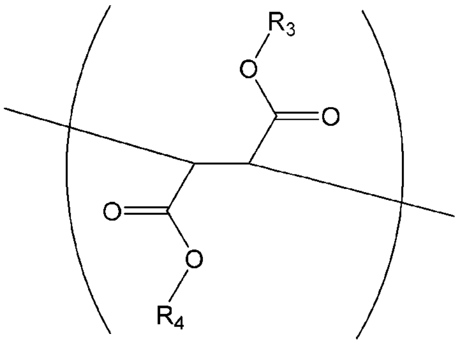

(3)

(In the formula, $R_3$ and $R_4$ each individually represent a linear alkyl group having 1 to 12 carbon atoms, a branched alkyl group having 3 to 12 carbon atoms or a cyclic alkyl group having 3 to 6 carbon atoms.)

[7] In the resin as described in [5] or [6], a weight average molecular weight (Mw) of a standard polystyrene equivalent obtained from an elution curve measured by gel permeation chromatography (GPC) is 50,000 or more.

[8] In the resin as described in any one of [5] to [7], a β relaxation temperature ($T_\beta$) during second scan temperature rise (temperature rise rate = 10°C /min) measured by differential scanning calorimetry (DSC) is 130°C or higher.

[9] A film includes the resin as described in any one of [5] to [8].

[10] In the film as described in [9], an out-of-plane retardation (Rth) measured at a wavelength of 589 nm represented by formula (a) below is -700 to 0 nm.

$$Rth = ((nx+ny)/2-nz) \times d \quad (a)$$

(In the formula, nx represents a refractive index in a fast axis direction (direction with smallest refractive index) in a film plane, ny represents a refractive index in a slow axis direction in a film plane, nz represents a refractive index in a perpendicular direction outside a film plane, and d represents a film thickness.)

[11] In the film as described in [9] or [10], a linear expansion coefficient α represented by formula (b) below is 95 ppm/°C or less.

$$\alpha = \Delta l / (\Delta T \times l) \quad (b)$$

(In the formula, Δl represents a film length change amount during temperature change, ΔT represents a film temperature change amount, and l represents a film length prior to temperature change.)

Effects of the Invention

According to the present disclosure, it is possible to provide a novel fumaric acid ester monomer that yields a resin having high $T_\beta$, a resin, and a film containing the same.

PREFERRED MODE FOR CARRYING OUT THE INVENTION

<Fumaric Acid Ester Monomer>

Hereinafter, a fumaric acid ester monomer which is an aspect of the present disclosure will be described in detail.

The present disclosure is the fumaric acid ester monomer (hereinafter also referred to as "monomer of present disclosure") represented by Formula (1) described below.

[Chem. 4]

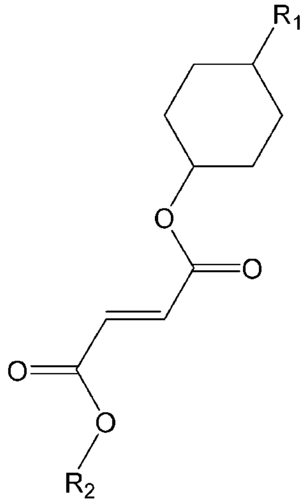

(1)

(In the formula, $R_1$ represents a linear alkyl group having 1 to 4 carbon atoms or a branched alkyl group having 3 to 5 carbon atoms. $R_2$ represents a linear alkyl group having 1 to 4 carbon atoms, a branched alkyl group having 3 to 12 carbon atoms, or a cyclic alkyl group having 3 to 6 carbon atoms.)

The β relaxation temperature ($T_\beta$) results from the side chain motion of the resin. In the monomer of the present disclosure, since rigid side chains containing cyclohexane rings having the $R_1$ group are introduced, the obtained resin has a high $T_\beta$. In particular, by introducing a cyclohexane ring having a $R_1$ group to only one end, it has a monomer melting point suited to polymerization, and thus high polymerization becomes possible, while maintaining high $T_\beta$. Thereby, a film containing resin obtained by polymerizing the monomer of the present disclosure is useful as a retardation film for which high thermal resistance is required.

As the linear alkyl group having 1 to 4 carbon atoms of $R_1$ in formula (1), a methyl group, ethyl group, propyl group and butyl group can be exemplified, as the branched alkyl group having 3 to 5 carbon atoms, an isopropyl group, isobutyl group, sec-butyl group, tert-butyl group, sec-pentyl group and tert-pentyl group can be exemplified. Since the resin obtained by polymerizing the monomer of the present disclosure increases in molecular weight while keeping high $T_\beta$, $R_1$ in formula (1) is preferably a methyl group, ethyl group, propyl group, isopropyl group, butyl group, tert-butyl group, isobutyl group, sec-butyl group, sec-pentyl group, and tert-pentyl group, and thereamong, a methyl group, ethyl group, isopropyl group and tert-butyl group are more preferable, an isopropyl group and a tert-butyl group are even more preferable, and a tert-butyl group is particularly preferable.

As the linear alkyl group having 1 to 4 carbon atoms of $R_2$ in formula (1), a methyl group, ethyl group, propyl group and butyl group can be exemplified, as the branched alkyl group having 3 to 12 carbon atoms, an isopropyl group, isobutyl group, sec-butyl group, sec-pentyl group, tert-pentyl group, sec-hexyl group and tert-hexyl group can be exemplified, and as the cyclic alkyl group having 3 to 6 carbon atoms, a cyclopropyl group, cyclopentyl group and cyclohexyl group can be exemplified. $R_2$ in formula (1) is preferably a methyl group, ethyl group, propyl group, isopropyl group, butyl group, isobutyl group, sec-butyl group, sec-pentyl group, tert-pentyl group, sec-hexyl group, tert-hexyl group, cyclopropyl group, cyclopentyl group or cyclohexyl group, and thereamong, an ethyl group, isopropyl group, sec-butyl group, cyclopentyl group or cyclohexyl group is more preferable, and an ethyl group, isopropyl group or cyclohexyl group is particularly preferable.

As fumaric acid ester monomers expressed by the general formula (1), (4-methylcyclohexyl)methyl fumarate, (4-methylcyclohexyl)ethyl fumarate, (4-methylcyclohexyl)propyl fumarate, (4-methylcyclohexyl)isopropyl fumarate, (4-methylcyclohexyl)butyl fumarate, (4-methylcyclohexyl)isobutyl fumarate, (4-methylcyclohexyl)sec-butyl fumarate, 4-methylcyclohexyl sec-pentyl fumarate, 4-methylcyclohexyl tert-pentyl fumarate, 4-methylcyclohexyl sec-hexyl fumarate, 4-methylcyclohexyl tert-hexyl fumarate, 4-methylcyclohexyl cyclopropyl fumarate, 4-methylcyclohexyl cyclopentyl fumarate, 4-methylcyclohexyl cyclohexyl fumarate, 4-ethylcyclohexyl methyl fumarate, (4-ethylcyclohexyl)ethyl fumarate, (4-ethylcyclohexyl)propyl fumarate, (4-ethylcyclohexyl)isopropyl fumarate, (4-ethylcyclohexyl)butyl fumarate, (4-ethylcyclohexyl)isobutyl fumarate, (4-ethylcyclohexyl)sec-butyl fumarate, (4-ethylcyclohexyl)sec-pentyl fumarate, (4-ethylcyclohexyl)tert-pentyl fumarate, (4-ethylcyclohexyl)sec-hexyl fumarate, (4-ethylcyclohexyl)tert-hexyl fumarate, (4-ethylcyclohexyl)cyclopropyl fumarate, (4-ethylcyclohexyl)cyclopentyl fumarate, (4-ethylcyclohexyl)cyclohexyl fumarate, (4-propylcyclohexyl)methyl fumarate, (4-propylcyclohexyl)ethyl fumarate, (4-propylcyclohexyl)propyl fumarate, (4-propylcyclohexyl)isopropyl fumarate, (4-propylcyclohexyl)butyl fumarate, (4-propylcyclohexyl)isobutyl fumarate, (4-propylcyclohexyl)sec-butyl fumarate, (4-propylcyclohexyl)sec-pentyl fumarate, (4-propylcyclohexyl)tert-pentyl fumarate, (4-propylcyclohexyl)sec-hexyl fumarate, (4-propylcyclohexyl)tert-hexyl fumarate, (4-propylcyclohexyl)cyclopropyl fumarate, (4-propylcyclohexyl)cyclopentyl fumarate, (4-propylcyclohexyl)cyclohexyl fumarate, (4-isopropylcyclohexyl)methyl fumarate, (4-isopropylcyclohexyl)ethyl fumarate, (4-isopropylcyclohexyl)propyl fumarate, (4-isopropylcyclohexyl)isopropyl fumarate, (4-isopropylcyclohexyl)butyl fumarate, (4-isopropylcyclohexyl)isobutyl fumarate, (4-isopropylcyclohexyl) sec-butyl fumarate, 4-isopropylcyclohexyl sec-pentyl fumarate, 4-isopropylcyclohexyl tert-pentyl fumarate, 4-isopropylcyclohexyl sec-hexyl fumarate, 4-isopropylcyclohexyl tert-hexyl fumarate, 4-isopropylcyclohexyl cyclopropyl fumarate, 4-isopropylcyclohexyl cyclopentyl fumarate, (4-isopropylcyclohexyl)cyclohexyl fumarate, (4-butylcyclohexyl)methyl fumarate, (4-butylcyclohexyl)ethyl fumarate, (4-butylcyclohexyl)propyl fumarate, (4-butylcyclohexyl)isopropyl fumarate, (4-butylcyclohexyl)butyl fumarate, (4-butylcyclohexyl)isobutyl fumarate, (4-butylcyclohexyl)sec-butyl fumarate, 4-butylcyclohexyl sec-pentyl fumarate, 4-butylcyclohexyl tert-pentyl fumarate, 4-butylcyclohexyl sec-hexyl fumarate, 4-butylcyclohexyl tert-hexyl fumarate, 4-butylcyclohexyl cyclopropyl fumarate, 4-butylcyclohexyl cyclopentyl fumarate, 4-butylcyclohexyl cyclohexyl fumarate, (4-tert-butylcyclohexyl)methyl fumarate, (4-tert-butylcyclohexyl)ethyl fumarate, (4-tert-butylcyclohexyl)propyl fumarate, (4-tert-butylcyclohexyl)isopropyl fumarate, (4-tert-butylcyclohexyl)butyl fumarate, (4-tert-butylcyclohexyl)isobutyl fumarate, (4-tert-butylcyclohexyl)sec-butyl fumarate, (4-tert-butylcyclohexyl)sec-pentyl fumarate, 4-tert-butylcyclohexyl tert-pentyl fumarate, 4-tert-butylcyclohexyl sec-hexyl fumarate, 4-tert-butylcyclohexyl tert-hexyl fumarate, 4-tert-butylcyclohexyl cyclopropyl fumarate, 4-tert-butylcyclohexyl cyclopentyl fumarate, 4-tert-butylcyclohexyl cyclohexyl fumarate, 4-isobutylcyclohexyl methyl fumarate, (4-isobutylcyclohexyl)ethyl fumarate, (4-isobutylcyclohexyl)propyl fumarate, (4-isobutylcyclohexyl)isopropyl fumarate, (4-isobutylcyclohexyl)butyl fumarate, (4-isobutylcyclohexyl)isobutyl fumarate, (4-isobutylcyclohexyl)sec-butyl fumarate, (4-isobutylcyclohexyl)sec-pentyl fumarate, (4-isobutylcyclohexyl)tert-pentyl fumarate, 4-isobutylcyclohexyl sec-hexyl fumarate, 4-isobutylcyclohexyl tert-hexyl fumarate, 4-isobutylcyclohexyl cyclopropyl fumarate, 4-isobutylcyclohexyl cyclopentyl fumarate, 4-isobutylcyclohexyl cyclohexyl fumarate, 4-sec-butylcyclohexyl methyl fumarate, 4-sec-butylcyclohexyl ethyl fumarate, 4-sec-butylcyclohexyl propyl fumarate, (4-sec-butylcyclohexyl)isopropyl fumarate, (4-sec-butylcyclohexyl)butyl fumarate, (4-sec-butylcyclohexyl)isobutyl fumarate, (4-sec-butylcyclohexyl)sec-butyl fumarate, (4-sec-butylcyclohexyl)sec-pentyl fumarate, (4-sec-butylcyclohexyl)tert-pentyl fumarate, (4-sec-butylcyclohexyl)sec-hexyl fumarate, (4-sec-butylcyclohexyl)tert-hexyl fumarate, (4-sec-butylcyclohexyl)cyclopropyl fumarate, (4-sec-butylcyclohexyl)cyclopentyl fumarate, (4-sec-butylcyclohexyl) cyclohexyl fumarate, (4-sec-pentylcyclohexyl)methyl fumarate, (4-sec-pentylcyclohexyl)ethyl fumarate, (4-sec-pentylcyclohexyl)propyl fumarate, (4-sec-pentylcyclohexyl)isopropyl fumarate, (4-sec-pentylcyclohexyl)butyl fumarate, (4-sec-pentylcyclohexyl)isobutyl fumarate, (4-sec-pentylcyclohexyl)sec-butyl fumarate, (4-sec-pentylcyclohexyl)sec-pentyl fumarate, (4-sec-pentylcyclohexyl)tert-pentyl fumarate, (4-sec-pentylcyclohexyl)sec-hexyl fumarate, (4-sec-pentylcyclohexyl)tert-hexyl fumarate, (4-sec-pentylcyclohexyl)cyclopropyl fumarate, (4-sec-pentylcyclohexyl)cyclopentyl fumarate, (4-sec-pentylcyclohexyl)cyclohexyl fumarate, (4-tert-pentylcyclohexyl)methyl fumarate, (4-tert-pentylcyclohexyl)ethyl fumarate, (4-tert-pentylcyclohexyl)propyl fumarate, (4-tert-pentylcyclohexyl)isopropyl fumarate, (4-tert-pentylcyclohexyl)butyl fumarate, (4-tert-pentylcyclohexyl)isobutyl fumarate, (4-tert-pentylcyclohexyl)sec-butyl fumarate, (4-tert-pentylcyclohexyl)sec-pentyl fumarate, 4-tert-pentylcyclohexyl fumarate tert-pentyl, 4-tert-pentylcyclohexyl sec-hexyl, 4-tert-pentylcyclohexyl tert-hexyl, 4-tert-pentylcyclohexyl cyclopropyl, 4-tert-pentylcyclohexyl cyclopentyl, and 4-tert-pentylcyclohexyl cyclohexyl fumarate can be exemplified. Due to the resins obtained by polymerizing the monomers of the present disclosure increasing in molecular weight while keeping high $T_\beta$, preferable thereamong are (4-methylcyclohexyl)ethyl fumarate, (4-methylcyclohexyl)isopropyl fumarate, (4-methylcyclohexyl)sec-butyl fumarate, (4-methylcyclohexyl)cyclopentyl fumarate, (4-methylcyclohexyl)cyclohexyl fumarate, (4-ethylcyclohexyl)ethyl fumarate, (4-ethylcyclohexyl)isopropyl fumarate, (4-ethylcyclohexyl)sec-butyl fumarate, (4-ethylcyclohexyl)cyclopentyl fumarate, (4-ethylcyclohexyl)cyclohexyl fumarate, (4-isopropylcyclohexyl)ethyl fumarate, (4-isopropylcyclohexyl)isopropyl fumarate, (4-isopropylcyclohexyl)sec-butyl fumarate, (4-isopropylcyclohexyl)cyclopentyl fumarate, (4-isopropylcyclohexyl)cyclohexyl fumarate, (4-tert-butylcyclohexyl)ethyl fumarate, (4-tert-butylcyclohexyl)isopropyl fumarate, (4-tert-butylcyclohexyl)sec-butyl fumarate, (4-tert-butylcyclohexyl)cyclopentyl fumarate, and (4-tert-butylcyclohexyl)cyclohexyl fumarate, and more preferable thereamong are (4-isopropylcyclohexyl)ethyl fumarate, (4-isopropylcyclohexyl)isopropyl fumarate, (4-isopropylcyclohexyl)sec-butyl fumarate, (4-isopropylcyclohexyl)cyclopentyl fumarate, (4-isopropylcyclohexyl)cyclohexyl fumarate, (4-tert-butylcyclohexyl)ethyl fumarate, (4-tert-butylcyclohexyl)isopropyl fumarate, (4-tert-butylcyclohexyl)sec-butyl fumarate, (4-tert-butylcyclohexyl)cyclopentyl fumarate and (4-tert-butylcyclohexyl)cyclohexyl fumarate; even more preferable are (4-tert-butylcyclohexyl)ethyl fumarate, (4-tert-butylcyclohexyl)isopropyl fumarate, (4-tert-butylcyclohexyl)sec-butyl fumarate, (4-tert-butylcyclohexyl)cyclopentyl fumarate and (4-tert-butylcyclohexyl)cyclohexyl fumarate; and particularly preferable are (4-tert-butylcyclohexyl)ethyl fumarate, (4-tert-butylcyclohexyl)isopropyl fumarate and (4-tert-butylcyclohexyl)cyclohexyl fumarate.

The monomer of the present disclosure has a structure in which the $R_1$ group, etc. bonds to the carbon atoms at the 1,4-positions of a cyclohexane ring. For this reason, in the monomer of the present disclosure, cis-trans isomers exist depending on the bond orientation of the $R_1$ group, etc. in the cyclohexane ring; however, it is not particularly limited to cis or trans isomer so long as a resin having a high $T_\beta$ is obtained.

In addition to the polymerization process construction of the monomer of the present disclosure being simple, since the obtained resin increases in molecular weight, the melting point of the monomer is preferably 80°C or less, more preferably 70°C or less, and particularly preferably 60°C or less. In the present disclosure, the melting point of the monomer is a value measured by the method described in the Examples described later.

In the present disclosure, as the synthesis method of the fumaric acid ester monomer represented by formula (1), it may be produced by any method so far as this fumaric acid ester monomer can be obtained, and, for example, the method represented by the below formula (b) and the below formula (c) can be exemplified.

[Chem. 5]

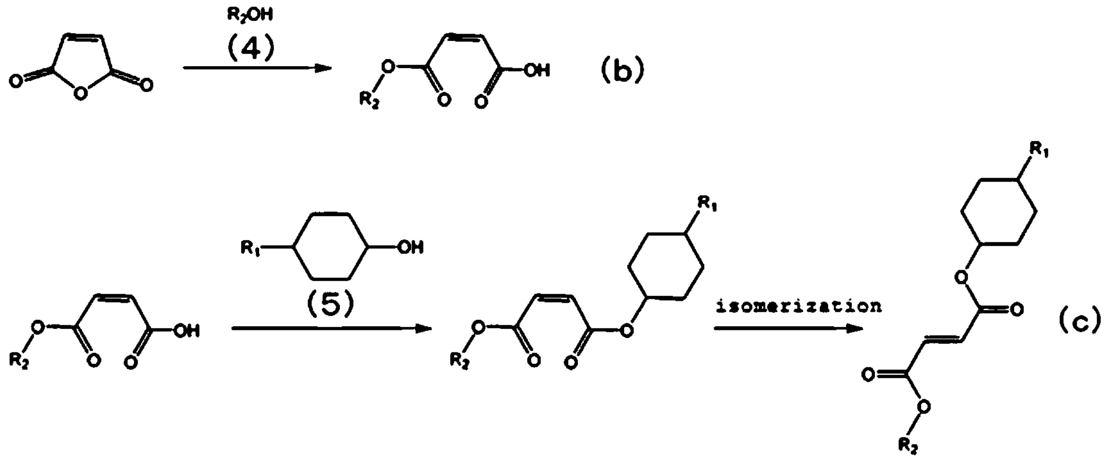


(1)

($R_2$ in formula (4) and $R_1$ in formula (5) have the same definitions as $R_1$ and $R_2$ in formula (1)).

Formula (b) is a maleic anhydride monoalkalyzation reaction of reacting the alcohol represented by the general formula (4) with maleic anhydride to synthesize a monoalkyl maleate.

$R_2$ of the alcohol represented by the general formula (4) is the same as $R_2$ of the general formula (1), and as specific alcohols, methanol, ethanol, propanol, isopropanol, butanol, isobutyl alcohol, 2-butanol, 2-pentanol, 2-methyl-2-butanol, 4-methyl-2-pentanol, 2-methyl-2-pentanol, cyclopropanol, cyclopentanol, cyclohexanol, etc. can be exemplified. Thereamong, ethanol, propanol, isopropanol, butanol, isobutyl alcohol, 2-butanol, 2-pentanol, 2-methyl-2-butanol, 4-methyl-2-pentanol, 2-methyl-2-pentanol, cyclopropanol, cyclopentanol and cyclohexanol are preferable, and thereamong, ethanol, isopropanol, 2-butanol, cyclopentanol and cyclohexanol are more preferable, and ethanol, isopropanol and cyclohexanol are particularly preferable.

The maleic anhydride monoalkylation reaction (formula (b)) is not particularly limited so long as monoalkylation is possible, and it can be reacted without catalyst or using an acid catalyst, and the reaction temperature is sufficient if selecting an appropriate temperature according to the substrate. As the acid catalyst, sulfuric acid, p-toluenesulfonic acid, xylenesulfonic acid, and ion exchange resins containing sulfo groups are preferable, and one or more of these can be used. Herein, as the ion exchange resins containing sulfo groups, Amberlite or Nafion can be exemplified. Since particularly high yield is obtained with these, sulfuric acid, p-toluenesulfonic acid and xylenesulfonic acid are more preferable.

The maleic anhydride monoalkylation reaction is not particularly limited so long as monoalkylation reaction is possible, and a solvent may or may not be used. The solvent used is not particularly limited so long as being a solvent which does not inhibit the reaction, for example, and halogen-based solvents such as dichloromethane and chloroform; ether-based solvents such as dioxane, tetrahydrofuran, diisopropyl ether, and cyclopentyl methyl ether; aromatic solvents such as benzene, toluene, xylene, chlorobenzene and dichlorobenzene; and hydrocarbon solvents such as cyclohexane and methylcyclohexane can be exemplified. These solvents may be used independently, or may be used by mixing two or more types.

The reaction temperature upon reacting maleic anhydride and alcohol is preferably 20 to 180°C, more preferably 25°C to 150°C, and particularly preferably 30 to 130°C, due to the monoalkyl maleate being obtained in high yield.

As the monoalkyl maleate obtained by the maleic anhydride monoalkylation reaction, monomethyl maleate, monoethyl maleate, monopropyl maleate, monobutyl maleate, monoisopropyl maleate, monoisobutyl maleate, mono-sec-butyl maleate, mono-sec-pentyl maleate, mono-tert-pentyl maleate, mono-sec-hexyl maleate, mono-tert-hexyl maleate, monocyclopropyl maleate, monocyclopentyl maleate and monocyclohexyl maleate can be exemplified. Thereamong, monoethyl maleate, monopropyl maleate, monoisopropyl maleate, monobutyl maleate, monoisobutyl maleate, mono-sec-butyl maleate, mono-sec-pentyl maleate, mono-tert-pentyl maleate, mono-sec-hexyl maleate, mono-tert-hexyl maleate, monocyclopropyl maleate, monocyclopentyl maleate and monocyclohexyl maleate are preferable, and thereamong, monoethyl maleate, monoisopropyl maleate, mono-sec-butyl maleate, monocyclopentyl maleate and monocyclohexyl maleate are more preferable, and monoethyl maleate, monoisopropyl maleate and monocyclohexyl maleate are particularly preferable.

Formula (c) is a method of synthesizing the fumaric acid ester represented by formula (1) by isomerization reaction, after condensation reaction of the monoalkyl maleate synthesized by formula (b) and the alcohol represented by general formula (5) under the presence of a catalyst.

$R_1$ in the alcohol represented by general formula (5) is the same as $R_1$ in general formula (1), and as specific alcohols, 4-methylcyclohexanol, 4-ethylcyclohexanol, 4-propylcyclohexanol, 4-isopropylcyclohexanol, 4-butylcyclohexanol, 4-tert-butylcyclohexanol, 4-isobutylcyclohexanol, 4-sec-butylcyclohexanol, 4-sec-pentylcyclohexanol, 4-tert-pentylcyclohexanol, etc. can be exemplified; thereamong, 4-methylcyclohexanol, 4-ethylcyclohexanol, 4-isopropylcyclohexanol and 4-tert-butylcyclohexanol are preferable, 4-isopropylcyclohexanol and 4-tert-butylcyclohexanol are more preferable, and 4-tert-butylcyclohexanol is particularly preferable.

The alcohol represented by general formula (5) has a structure in which a hydroxyl group and $R_1$ group are bonded to the carbon atoms at the 1,4-positions of the cyclohexane ring. For this reason, according to the bonding direction of the hydroxy group and $R_1$ group to the cyclohexane ring in the alcohol represented by general formula (5), cis-trans isomers exist; however, so long as a resin having high $T_\beta$ is obtained, it is not particularly limited to the cis isomer or trans isomer.

The catalyst is not particularly limited so long as the condensation reaction is possible, and a condensing agent or acid catalyst can be used. As the condensing agent, for example, 1-[3-(dimethylamino)propyl]-3-ethylcarbodiimide, 1-[3-(dimethylamino)propyl]-3-ethylcarbodiimide hydrochloride, N,N'-diisopropylcarbodiimide, N,N'-dicyclohexylcarbodiimide, bis(2,6-diisopropylphenyl)carbodiimide, bis(trimethylsilyl)carbodiimide, 1-cyclohexyl-3-(2-morpholinoethyl)carbodiimide-p-toluenesulfonate, N,N'-di-tert-butylcarbodiimide, and 1-[3-(dimethylamino)propyl]-3-ethylcarbodiimide methiodide can be exemplified, and thereamong, 1-[3-(dimethylamino)propyl]-3-ethylcarbodiimide, 1-[3-(dimethylamino)propyl]-3-ethylcarbodiimide hydrochloride, N,N'-diisopropylcarbodiimide, and N,N'-dicyclohexylcarbodiimide are preferable. Two or more types of these ester bond forming condensing agents may be used.

As the acid catalyst, sulfuric acid, p-toluenesulfonic acid, xylenesulfonic acid, and ion exchange resins containing sulfo groups are preferably, and one or more of these can be used. Herein, as the ion exchange resins containing sulfo groups, Amberlite or Nafion can be exemplified. Since particularly high yield is obtained, thereamong, sulfuric acid, p-toluenesulfonic acid and xylenesulfonic acid are more preferable.

In the condensation reaction under the presence of a condensing agent of the monoalkyl maleate synthesized by formula (b) and alcohol, it is preferable to conduct by adding a base due to the reaction progressing smoothly. As the base used, for example, organic bases can be exemplified, and as the organic bases, triethylamine, tributylamine, diisopropylethylamine, pyridine, 4-dimethylaminopyridine, piperidine, piperazine, pyrrolidine, morpholine, N-methylmorpholine, imidazole, and N-methylimidazole can be exemplified.

The isomerization reaction is not particularly limited so long as capable of isomerization, and there are methods using an isomerization catalyst. As the isomerization catalyst, for example, amines such as dimethylamine, diethylamine, di-n-propylamine, diisopropylamine, di-n-butylamine, di-sec-butylamine, di-tert-butylamine, dicyclohexylamine, aziridine, pyrrolidine, morpholine, piperazine, piperidine and ethylenediamine are preferable, and piperidine and ethylenediamine are particularly preferable.

The solvent upon reacting the monoalkyl maleate and alcohol is not particularly limited so long as being a solvent which does not inhibit the reaction, for example, and halogen-based solvents such as dichloromethane and chloroform; ether-based solvents such as dioxane, tetrahydrofuran, diisopropyl ether, and cyclopentyl methyl ether; aromatic solvents such as benzene, toluene, xylene, chlorobenzene and dichlorobenzene; and hydrocarbon solvents such as cyclohexane and methylcyclohexane can be exemplified. These solvents may be used independently, or may be used by mixing two or more types thereof.

The solvent upon performing the isomerization reaction is not particularly limited so long as not reacting with the amine and, for example, halogen-based solvents such as dichloromethane and chloroform; ether-based solvents such as dioxane, tetrahydrofuran, diisopropyl ether, and cyclopentyl methyl ether; aromatic hydrocarbons such as toluene and xylene; etc. can be exemplified.

<Resin>

Hereinafter, a resin which is one aspect of the present disclosure will be described in detail.

The present disclosure is a resin containing a residue unit (hereinafter also referred to as "resin of present disclosure") represented by the below structural formula (2) derived from the fumaric acid ester monomer represented by

formula (1).

[Chem. 6]

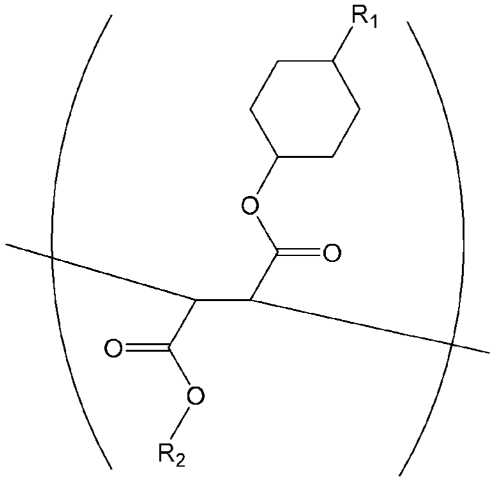

(2)

(In the formula, $R_1$ represents a linear alkyl group having 1 to 4 carbon atoms or a branched alkyl group having 3 to 5 carbon atoms. $R_2$ represents a linear alkyl group having 1 to 4 carbon atoms, a branched alkyl group having 3 to 12 carbon atoms, or a cyclic alkyl group having 3 to 6 carbon atoms.)

$R_1$ and $R_2$ in formula (2) can use the same ones as $R_1$ and $R_2$ in formula (1).

As the specific residue units expressed by general formula (2), (4-methylcyclohexyl) fumarate methyl residue, (4-methylcyclohexyl)ethyl fumarate residue, (4-methylcyclohexyl)propyl fumarate residue, (4-methylcyclohexyl)isopropyl fumarate residue, (4-methylcyclohexyl)butyl fumarate residue, (4-methylcyclohexyl)isobutyl fumarate residue, (4-methylcyclohexyl)sec-butyl fumarate residue, (4-methylcyclohexyl)sec-pentyl fumarate residue, 4-methylcyclohexyl tert-pentyl fumarate residue, 4-methylcyclohexyl sec-hexyl fumarate residue, 4-methylcyclohexyl tert-hexyl fumarate residue, 4-methylcyclohexyl cyclopropyl fumarate residue, 4-methylcyclohexyl cyclopentyl fumarate residue, 4-methylcyclohexyl cyclohexyl fumarate residue, 4-ethylcyclohexyl methyl fumarate residue, (4-ethylcyclohexyl) ethyl fumarate residue, (4-ethylcyclohexyl) propyl fumarate residue, (4-ethylcyclohexyl) isopropyl fumarate residue, (4-ethylcyclohexyl) butyl fumarate residue, (4-ethylcyclohexyl) isobutyl fumarate residue, (4-ethylcyclohexyl) sec-butyl fumarate residue, (4-ethylcyclohexyl) sec-pentyl fumarate residue, (4-ethylcyclohexyl) tert-pentyl fumarate residue, 4-ethylcyclohexyl sec-hexyl fumarate residue, 4-ethylcyclohexyl tert-hexyl fumarate residue, 4-ethylcyclohexyl cyclopropyl fumarate residue, 4-ethylcyclohexyl cyclopentyl fumarate residue, 4-ethylcyclohexyl cyclohexyl fumarate residue, 4-propylcyclohexyl methyl fumarate residue, 4-propylcyclohexyl ethyl fumarate residue, 4-propylcyclohexyl propyl fumarate residue, (4-propylcyclohexyl) isopropyl fumarate residue, (4-propylcyclohexyl) butyl fumarate residue, (4-propylcyclohexyl) isobutyl fumarate residue, sec-butyl fumarate (4-propylcyclohexyl) residue, (4-propylcyclohexyl) sec-pentyl fumarate residue, (4-propylcyclohexyl) tert-pentyl fumarate residue, (4-propylcyclohexyl) sec-hexyl fumarate residue, (4-propylcyclohexyl) tert-hexyl fumarate residue, (4-propylcyclohexyl) cyclopropyl fumarate residue, (4-propylcyclohexyl) cyclopentyl fumarate residue, (4-propylcyclohexyl) cyclohexyl fumarate residue, (4-isopropylcyclohexyl) methyl fumarate residue, (4-isopropylcyclohexyl) ethyl fumarate residue, (4-isopropylcyclohexyl) propyl fumarate residue, (4-isopropylcyclohexyl) isopropyl fumarate residue, (4-isopropylcyclohexyl) butyl fumarate residue, (4-isopropylcyclohexyl) isobutyl fumarate residue, (4-isopropylcyclohexyl) sec-butyl fumarate residue, 4-isopropylcyclohexyl sec-pentyl fumarate residue, 4-isopropylcyclohexyl tert-pentyl fumarate residue, 4-isopropylcyclohexyl sec-hexyl fumarate residue, 4-isopropylcyclohexyl tert-hexyl fumarate residue, 4-isopropylcyclohexyl cyclopropyl fumarate residue, (4-isopropylcyclohexyl) cyclopentyl fumarate residue, (4-isopropylcyclohexyl) cyclohexyl fumarate residue, (4-butylcyclohexyl) methyl fumarate residue, (4-butylcyclohexyl) ethyl fumarate residue, (4-butylcyclohexyl) propyl fumarate residue, (4-butylcyclohexyl) isopropyl fumarate residue, (4-butylcyclohexyl) butyl fumarate residue, (4-butylcyclohexyl) isobutyl fumarate residue, (4-butylcyclohexyl) sec-butyl fumarate residue, (4-butylcyclohexyl) sec-pentyl fumarate residue, (4-butylcyclohexyl) tert-pentyl fumarate residue, (4-butylcyclohexyl) sec-hexyl fumarate residue, (4-butylcyclohexyl) tert-hexyl fumarate residue, (4-butylcyclohexyl) cyclopropyl fumarate residue, (4-butylcyclohexyl) cyclopentyl fumarate residue, (4-butylcyclohexyl) cyclohexyl fumarate residue, (4-tert-butylcyclohexyl) methyl fumarate residue, (4-tert-butylcyclohexyl) ethyl fumarate residue, (4-tert-butylcyclohexyl) propyl fumarate residue, (4-tert-butylcyclohexyl) isopropyl fumarate residue, (4-tert-butylcyclohexyl) butyl fumarate residue, (4-tert-butylcyclohexyl) isobutyl fumarate residue, 4-tert-butylcyclohexyl sec-butyl fumarate residue, 4-tert-butylcyclohexyl sec-pentyl fumarate residue, 4-tert-butylcyclohexyl tert-pentyl fumarate residue, 4-tert-butylcyclohexyl sec-hexyl fumarate residue, 4-tert-butylcyclohexyl tert-hexyl fumarate residue, 4-tert-butylcyclohexyl cyclopropyl fumarate residue, (4-tert-butylcyclohexyl) cyclopentyl fumarate residue, (4-tert-butylcyclohexyl) cyclohexyl fumarate residue, (4-isobutylcyclohexyl) methyl fumarate residue, (4-isobutylcyclohexyl) ethyl fumarate residue, (4-isobutylcyclohexyl) propyl fumarate residue, (4-isobutylcyclohexyl) isopropyl fumarate residue, 4-isobutylcyclohexyl butyl fumarate residue, 4-isobutylcyclohexyl isobutyl fumarate residue, 4-isobutylcyclohexyl sec-butyl fumarate residue, 4-isobutylcyclohexyl sec-pentyl fumarate residue, 4-isobutylcyclohexyl tert-pentyl fumarate residue, 4-isobutylcyclohexyl sec-hexyl fumarate residue, 4-isobutylcyclohexyl tert-hexyl fumarate residue, (4-isobutylcyclohexyl) cyclopropyl fumarate residue, (4-isobutylcyclohexyl) cyclopentyl fumarate residue, (4-isobutylcyclohexyl) cyclohexyl fumarate residue, (4-sec-butylcyclohexyl) methyl fumarate residue, (4-sec-butylcyclohexyl) ethyl fumarate residue, (4-sec-butylcyclohexyl) propyl fumarate residue, (4-sec-butylcyclohexyl) isopropyl fumarate residue, (4-sec-butylcyclohexyl) butyl fumarate residue, (4-sec-butylcyclohexyl) isobutyl fumarate residue, (4-sec-butylcyclohexyl) sec-butyl fumarate residue, (4-sec-butylcyclohexyl) sec-pentyl fumarate residue, (4-sec-butylcyclohexyl) tert-pentyl fumarate residue, (4-sec-butylcyclohexyl) sec-hexyl fumarate residue, (4-sec-butylcyclohexyl) tert-hexyl fumarate residue, (4-sec-butylcyclohexyl) cyclopropyl fumarate residue, (4-sec-butylcyclohexyl) cyclopentyl fumarate residue, (4-sec-butylcyclohexyl) cyclohexyl fumarate residue, (4-sec-pentylcyclohexyl) methyl fumarate residue, (4-sec-pentylcyclohexyl) ethyl fumarate residue, (4-sec-pentylcyclohexyl) propyl fumarate residue, (4-sec-pentylcyclohexyl) isopropyl fumarate residue, (4-sec-pentylcyclohexyl) butyl fumarate residue, (4-sec-pentylcyclohexyl) isobutyl fumarate residue, (4-sec-pentylcyclohexyl) sec-butyl fumarate residue, (4-sec-pentylcyclohexyl) sec-pentyl fumarate residue, (4-sec-pentylcyclohexyl) tert-pentyl fumarate residue, (4-sec-pentylcyclohexyl) sec-hexyl fumarate residue, (4-sec-pentylcyclohexyl) tert-hexyl fumarate residue, (4-sec-pentylcyclohexyl) cyclopropyl fumarate residue, (4-sec-pentylcyclohexyl) cyclopentyl fumarate residue, (4-sec-pentylcyclohexyl)cyclohexyl fumarate residue, (4-tert-pentylcyclohexyl)methyl fumarate residue, (4-tert-pentylcyclohexyl)ethyl fumarate residue, (4-tert-pentylcyclohexyl)propyl fumarate residue, (4-tert-pentylcyclohexyl)isopropyl fumarate residue, (4-tert-pentylcyclohexyl)butyl fumarate residue, (4-tert-pentylcyclohexyl) isobutyl fumarate residue, (4-tert-pentylcyclohexyl) sec-butyl fumarate residue, (4-tert-pentylcyclohexyl) sec-pentyl fumarate residue, (4-tert-pentylcyclohexyl) tert-pentyl fumarate residue, (4-tert-pentylcyclohexyl) sec-hexyl fumarate residue, (4-tert-pentylcyclohexyl) tert-hexyl fumarate residue, (4-tert-pentylcyclohexyl) cyclopropyl fumarate residue, (4-tert-pentylcyclohexyl) cyclopentyl fumarate residue, and (4-tert-pentylcyclohexyl) cyclohexyl fumarate residue can be exemplified. Due to the resins obtained by polymerizing the monomers of the present disclosure increasing in molecular weight while keeping high $T_\beta$, preferable thereamong are (4-methylcyclohexyl) ethyl fumarate residue, (4-methylcyclohexyl) isopropyl fumarate residue, (4-methylcyclohexyl) sec-butyl fumarate residue, (4-methylcyclohexyl) cyclopentyl fumarate residue, (4-methylcyclohexyl) cyclohexyl fumarate residue, (4-ethylcyclohexyl) ethyl fumarate residue, (4-ethylcyclohexyl) isopropyl fumarate residue, 4-ethylcyclohexyl sec-butyl fumarate residue, 4-ethylcyclohexyl cyclopentyl fumarate residue, 4-ethylcyclohexyl cyclohexyl fumarate residue, 4-isopropylcyclohexyl ethyl fumarate residue, 4-isopropylcyclohexyl isopropyl fumarate residue, 4-isopropylcyclohexyl sec-butyl fumarate residue, 4-isopropylcyclohexyl cyclopentyl fumarate residue, (4-isopropylcyclohexyl) cyclohexyl fumarate residue, (4-tert-butylcyclohexyl) ethyl fumarate residue, (4-tert-butylcyclohexyl) isopropyl fumarate residue, (4-tert-butylcyclohexyl) sec-butyl fumarate residue, (4-tert-butylcyclohexyl) cyclopentyl fumarate residue and (4-tert-butylcyclohexyl) cyclohexyl fumarate residue; more preferably thereamong are (4-isopropylcyclohexyl) ethyl fumarate residue, (4-isopropylcyclohexyl) isopropyl fumarate residue, (4-isopropylcyclohexyl) sec-butyl fumarate residue, (4-isopropylcyclohexyl) cyclopentyl fumarate residue, (4-isopropylcyclohexyl) cyclohexyl fumarate residue, (4-tert-butylcyclohexyl) ethyl fumarate residue, (4-tert-butylcyclohexyl) isopropyl fumarate residue, (4-tert-butylcyclohexyl) sec-butyl fumarate residue, (4-tert-butylcyclohexyl) cyclopentyl fumarate residue and (4-tert-butylcyclohexyl) cyclohexyl fumarate residue, even more preferably are (4-tert-butylcyclohexyl) ethyl fumarate residue, (4-tert-butylcyclohexyl) isopropyl fumarate residue, (4-tert-butylcyclohexyl) sec-butyl fumarate residue, (4-tert-butylcyclohexyl) cyclopentyl fumarate residue and (4-tert-butylcyclohexyl) cyclohexyl fumarate residue, and particularly preferable are (4-tert-butylcyclohexyl)ethyl fumarate residue, (4-tert-butylcyclohexyl)isopropyl fumarate residue and (4-tert-butylcyclohexyl)cyclohexyl fumarate residue.

The residue unit represented by formula (2) has a structure in which the $R_1$ group, etc. are bonded to carbon atoms at the 1,4-positions of the cyclohexane ring. For this reason, in the residue unit represented by formula (2), cis-trans isomers will exist depending on the bonding direction of the $R_1$ group, etc. relative to the cyclohexane ring; however, it is not particularly limited to cis or trans so long as a resin having a high $T_\beta$ is obtained. The resin of the present disclosure may have a residue unit represented by formula (2) of the cis isomer and a residue unit represented by formula (2) of the trans isomer, or may only have either one of the residue units.

Due to forming a resin of high heat resistance derived from the high $T_\beta$, a fumaric acid ester resin containing 40 mol% or more, preferably 50 mol% or more, particularly preferably 60 mol% or more, and more preferably 80 mol% or more of a fumaric acid ester residue unit represented by formula (2) can be exemplified as the resin of the present disclosure. In the resin of the present disclosure, the upper limit for the ratio of residue units represented by formula (2) is not particularly limited, and may be 100 mol%; however, for example, it may be 95 mol% or less, or 90 mol% or less.

Since the resin of the present disclosure has favorable heat resistance and polymerization property, in addition to

the residue unit represented by structural formula (2), a residue unit represented by the structural formula (3) below may be further contained.

[Chem. 7]

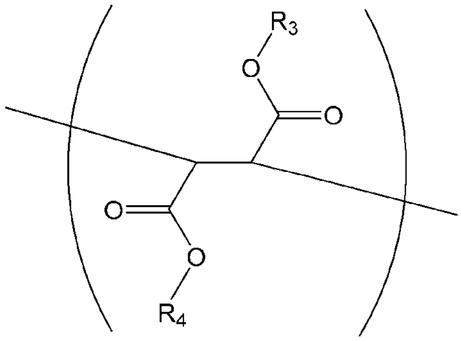

(3)

(In the formula, $R_3$ and $R_4$ each individually represent a linear alkyl group having 1 to 12 carbon atoms, a branched alkyl group having 3 to 12 carbon atoms or a cyclic alkyl group having 3 to 6 carbon atoms.)

In formula (3), $R_3$ and $R_4$ each individually represent a linear alkyl group having 1 to 12 carbon atoms, a branched alkyl group having 3 to 12 carbon atoms or a cyclic alkyl group having 3 to 6 carbon atoms.

As specific $R_3$ and $R_4$, for example, an ethyl group, propyl group, isopropyl group, butyl group, isobutyl group, sec-butyl group, tert-butyl group, sec-pentyl group, tert-pentyl group, sec-hexyl group, tert-hexyl group, cyclopropyl group, cyclopentyl group, and cyclohexyl group can be exemplified, thereamong, an ethyl group, isopropyl group, sec-butyl group, tert-butyl group, cyclopentyl group, and cyclohexyl group are preferred due to making films superior in heat resistance and mechanical characteristics, and an isopropyl group is particularly preferable.

Herein, as the fumaric acid ester residue unit represented by formula (3), specifically, diethyl fumarate residue, diisopropyl fumarate residue, di-sec-butyl fumarate residue, di-tert-butyl fumarate residue, di-sec-pentyl fumarate residue, di-tert-pentyl fumarate residue, di-sec-hexyl fumarate residue, di-tert-hexyl fumarate residue, dicyclopropyl fumarate residue, dicyclopentyl fumarate residue, dicyclohexyl fumarate residue, or residues substituting $R_3$ or $R_4$ in these residues (for example, ethyl isopropyl fumarate residue, ethyl sec-butyl fumarate residue, etc.) or the like can be exemplified. Preferable thereamong are diethyl fumarate residue, diisopropyl fumarate residue, di-sec-butyl fumarate residue, di-tert-butyl fumarate residue, dicyclopentyl fumarate residue, dicyclohexyl fumarate residue, isopropylethyl fumarate residue, isopropyl-tert-butyl fumarate residue, cyclohexylethyl fumarate residue, cyclohexylisopropyl fumarate residue, cyclohexyl-sec-butyl fumarate residue, cyclohexyl-tert-butyl fumarate residue, cyclohexyl sec-pentyl fumarate residue, cyclohexyl tert-pentyl fumarate residue, cyclohexyl sec-hexyl fumarate residue, cyclohexyl tert-hexyl fumarate residue, cyclohexyl cyclopentyl fumarate, etc., particularly preferable are diethyl fumarate residue, diisopropyl fumarate residue, di-sec-butyl fumarate residue, di-tert-butyl fumarate residue, dicyclopentyl fumarate residue and dicyclohexyl fumarate residue, and even more preferable are diethyl fumarate residue and diisopropyl fumarate residue. These may be independent or two or more types may be combined.

In the case of the resin of the present disclosure containing the residue unit represented by formula (3), the ratio thereof is preferably 5 mol% or more, and is more preferably 10 mol% or more. In addition, the above ratio is 60 mol% or less, more preferably 50 mol% or less, even more preferably 40 mol% or less, and particularly preferably 20 mol% or less.

Due to making a film superior in heat resistance and mechanical characteristics, a fumaric acid ester resin containing, for example, 40 mol% or more, more preferably 50 mol% or more, particularly preferably 60 mol% or more, and even more preferably 80 mol% or more of a total of the fumaric acid ester residue unit represented by formula (2) and the fumaric acid ester residue unit represented by formula (3) can be exemplified as the resin of the present disclosure.

The above-mentioned fumaric acid ester resin may contain residue units other than the residue units represented by formulas (2) and (3). As residue units other than the residue units represented by formulas (2) and (3), for example, one, two or more types of styrenic residues such as styrene residue and $\alpha$-methylstyrene residue; acrylic acid residues; acrylic ester residues such as methyl acrylate residues, ethyl acrylate residues and butyl acrylate residues; methacrylic acid residues; methacrylate ester residues such as methyl methacrylate residue, ethyl methacrylate residue and butyl methacrylate residue; vinyl ester residues such as vinyl acetate residue and vinyl propionate residue; acrylonitrile residue; methacrylonitrile residue; and olefinic residues such as ethylene residue and propylene residue can be exemplified.

Due to making a product superior particularly in heat resistance, the $\beta$ relaxation temperature ($T_\beta$) of the resin of the present disclosure during second scan temperature rise (temperature rise rate = 10°C /min) measured by differential

scanning calorimetry (DSC) is preferably 130°C or more, more preferably 130°C to 300°C, even more preferably 140°C to 250°C, and particularly preferably 150°C to 200°C.

Due to making a product particularly superior in mechanical characteristics, the standard polystyrene equivalent weight average molecular weight (Mw) of the resin of the present disclosure obtained from the elution curve measured by gel permeation chromatography (GPC) is preferably 50,000 or more, more preferably 80,000 or more, even more preferably 80,000 to 5,000,000, particularly preferably 200,000 to 2,000,000, more particularly preferably 300,000 to 1,000,000, and most preferably 400,000 to 800,000.

As the production method of the resin of the present disclosure, it may be produced by any method so long as this resin is obtained and, for example, a method radical polymerizing the fumaric acid ester monomer represented by formula (1), or a method performing transesterification on the fumaric acid ester resin can be exemplified, and thereamong, a method radical polymerizing the fumaric acid ester monomer represented by formula (1) is preferable.

As the method of radical polymerization, for example, any of bulk polymerization, solution polymerization, suspension polymerization, precipitation polymerization, emulsion polymerization or the like can be adopted. Thereamong, bulk polymerization or suspension polymerization is preferable due to a high molecular weight fumaric acid ester resin being obtained.

The polymerization temperature at which performing radical polymerization is not particularly limited so long as at least a temperature at which the fumaric acid ester monomer melts; however, a lower temperature is preferable due to particularly high molecular weight fumaric acid ester resin being obtained. For example, it is preferably 120°C or less, more preferably 100°C or less, and particularly preferably 80°C or less.

As a polymerization initiator upon performing the radical polymerization, for example, organic peroxides such as benzoyl peroxide, lauryl peroxide, octanoyl peroxide, acetyl peroxide, di-tert-butyl peroxide, tert-butylcumyl peroxide, dicumyl peroxide, tert-butyl peroxyacetate, tert-butyl peroxybenzoate, 2,5-dimethyl-2,5-di(2-ethylhexanoylperoxy)hexane, tert-butyl peroxypivalate, tert-butyl perhydroxyoxide, and tert-butylperoxy-2-ethylhexanoate; azo initiators such as 2,2'-azobis(2,4-dimethylvaleronitrile), 2,2'-azobis(2-methylpropionate)dimethyl, 2,2'-azobis(2-butyronitrile), 2,2'-azobisisobutyronitrile, dimethyl-2'-azobisisobutyrate, and 1,1'-azobis(cyclohexane-1-carbonitrile); etc. can be exemplified.

<Film>

Hereinafter, a film which is an aspect of the present disclosure will be described in detail.

The resin of the present disclosure can be favorably used as the film for optical components. In particular, since the $T_\beta$ of the resin is high, it can be used as a film superior in heat resistance.

In the film of the present disclosure, the out-of-plane retardation (Rth) measured at a wavelength of 589 nm represented by formula (a) below is preferably -700 to 0 nm due to making a film superior in viewing angle characteristics, is more preferably -240 to -20 nm, and particularly preferably -160 to -30 nm. In the present disclosure, the out-of-plane retardation of the film is a value measured by the measurement method of the Examples described later.

$$Rth=[(nx+ny)/2-nz]\,d \quad (a)$$

(In the formula, nx indicates the refractive index in the fast axis direction in the film plane, ny indicates the refractive index in the slow axis direction in the film plane, nz indicates the refractive index in the perpendicular direction outside the film plane, and d indicates the thickness (nm) of the film.)

In the film, from the viewpoint of suitability to film thinning of optical components, the thickness is preferably 200.0 μm or less, is more preferably 0.1 to 80.0 μm, and particularly preferably 0.1 to 50.0 μm.

Since the film of the present disclosure contains a fumaric acid ester resin having a high $T_\beta$, a film having low linear expansion coefficient and superior in dimensional stability when at high temperature can be obtained.

In the film of the present disclosure, the linear expansion coefficient $\alpha$ represented by formula (b) below is preferably 95 ppm/°C or less, is more preferably 0 to 90 ppm/°C, is even more preferably 10 to 85 ppm/°C, and is particularly preferably 15 to 80 ppm/°C, due to making a film superior in dimensional stability when at high temperature. In the present disclosure, the linear expansion coefficient of the film, for example, is a value measured by the measurement method in the Examples described later.

$$\alpha=\Delta l/(\Delta T \times l) \quad (b)$$

(In the formula, $\Delta l$ indicates the film length change amount during temperature change, $\Delta T$ indicates the film temperature change amount, and l indicates the film length prior to temperature change.)

The film may contain an antioxidant in order to improve the thermal stability. As this antioxidant, for example, hindered phenol-based antioxidants, phosphorus-based antioxidants, sulfur-based antioxidants, lactone-based antioxidants, amine-based antioxidants, hydroxylamine-based antioxidants, vitamin E-based antioxidants and other antioxidants can be exemplified, and these antioxidants may each be independent, or two or more types may be combined.

The film may contain a hindered amine-based light stabilizer or ultraviolet absorber to enhance weather resistance. As the ultraviolet absorber, for example, benzotriazoles, benzophenones, triazines, benzoates and the like can be exemplified.

The film may contain other polymers, surfactants, polyelectrolytes, conductive complexes, pigments, dyes, antistatic agents, antiblocking agents, lubricants, etc. in ranges not exceeding the gist of the invention.

The production method of the film is not particularly limited, for example, and the resin which is the raw material can be manufactured by forming a long film by a method such as solution casting.

Herein, solution casting is a method which obtains a film by casting a resin solution (generally called dope) onto a support substrate, then evaporating the solvent by heating and drying, and peeling from the substrate.

The solvent used in the resin solution of the solution casting method may be any solvent so long as being a solvent which can dissolve the resin, etc., and the boiling point of the solvent is preferably 200°C or less, and more preferably 170°C or less so that residual solvent hardly remains upon obtaining the film.

As the solvent, for example, halogenated hydrocarbons such as chloroform, dichloromethane, carbon tetrachloride, dichloroethane, tetrachloroethane, trichloroethylene, tetrachloroethylene, chlorobenzene, and dichlorobenzene; phenols such as phenol and chlorophenol; aromatic hydrocarbons such as benzene, toluene, xylene, methoxybenzene, mesitylene, and dimethoxybenzene; ketone solvents such as acetone, methyl ethyl ketone, methyl isobutyl ketone, cyclohexanone, cyclopentanone, 2-pyrrolidone, and N-methyl-2-pyrrolidone; ester solvents such as ethyl acetate and butyl acetate; alcohol-based solvents such as t-butyl alcohol, glycerin, ethylene glycol, triethylene glycol, ethylene glycol monomethyl ether, diethylene glycol dimethyl ether, propylene glycol, dipropylene glycol, and 2-methyl-2,4-pentanediol; amide solvents such as dimethylformamide and dimethylacetamide; nitrile solvents such as acetonitrile and butyronitrile; ether solvents such as diethyl ether, dibutyl ether, and tetrahydrofuran; and carbon disulfide, ethyl cellosolve, butyl cellosolve or the like alone or by mixing can be exemplified.

The viscosity of the resin solution can be adjusted by the molecular weight of each component, concentration and type of solvent. The viscosity of the resin solution is not particularly limited; however, to further facilitate film casting, it is preferably 100 to 30,000 cps, more preferably 300 to 20,000 cps, and particularly preferably 300 to 15,000 cps.

In the present disclosure, the concentration of the raw material resin in the dope is not particularly limited so long as dissolution and film production are possible. The method for carrying out dissolution may be carried out so as to make a predetermined concentration in the stage of dissolution, and may adjust to a predetermined high concentration solution in a concentration step after preparing as a low concentration solution in advance. Furthermore, after establishing a resin solution of high concentration in advance, a resin solution of a predetermined low concentration may be established by adding various additives.

In addition, the support substrates that can be used are not particularly limited and, for example, polymeric substrates made from polyesters such as polyethylene terephthalate and polyethylene naphthalate, and polycarbonates, polystyrene, polyethylene, polypropylene, polyacrylic, polyvinyl chloride or polyvinylidene chloride, celluloses such as cellulose acetate and cellulose ether, polyvinyl alcohol, polyamide, polyimide, polyarylate, polysulfone, polyethersulfone, polyetherketone, phenolic resin, epoxy resin, alicyclic polyolefin, norbornene-based thermoplastic transparent resin; glass substrates such as glass plate or quartz substrate; metal substrates such as aluminum, stainless steel, and ferrotype; inorganic substrates such as ceramic substrates, etc. can be exemplified. The above-mentioned substrate is preferably a polymeric substrate of a polyester such as polyethylene terephthalate and polyethylene naphthalate, polypropylene, polyacrylic, a cellulose such as cellulose acetate and cellulose ether, polyimide, alicyclic polyolefin, norbornene-based thermoplastic transparent resin, or the like. It is more preferably a polymeric substrate of a polyester such as polyethylene terephthalate and polyethylene naphthalate, polypropylene, polyimide, alicyclic polyolefin, norbornene-based thermoplastic transparent resin, or the like.

The casting method is not particularly limited, and a known method can be adopted. For example, the T-die method, doctor blade method, bar coater method, slot die method, lip coater method, reverse gravure coating method, microgravure method, spin coating method, brush coating method, roll coating method, flexographic printing method, etc. can be exemplified.

The drying method in the drying step is not particularly limited, and a known heating means can be adopted. For example, a hot air blower, heating roller, far infrared heater or the like can be exemplified.

The drying temperature is preferably 30 to 200°C, and is particularly preferably 40 to 160°C. It should be noted that it is not a problem even under a condition of only one stage drying temperature, and for maintaining external appearance and shortening drying time, is not a problem even with multistage drying such that dries at low temperature in a first stage, and dries at high temperature in second and later stages.

As the peeling rate of the film in the substrate peeling step, the range of 0.1 to 30 m/min can be exemplified due to the productivity, mechanical precision and stability, and more preferably, the range of 1 to 30 m/min can be exemplified.

The film can be further laminated with a film containing another resin as necessary. As the other resin, for

example, polyethersulfone, polyarylate, polyethylene terephthalate, polynaphthalene terephthalate, polycarbonate, cyclic polyolefin, maleimide resin, fluorine resin, polyimide, etc. can be exemplified. In addition, a liquid crystal layer and hard coat layer, gas barrier layer, and layer having controlled refractive index (low reflection layer) can also be laminated.

The film of the present disclosure has superior heat resistance and optical characteristics, and thus can be suitably used in a retardation film used in applications such as for liquid crystal display devices, and organic EL display devices.

In addition, by arranging the film of the present disclosure on at least one surface of a polarizer, it is possible to obtain a polarizing plate superior in heat resistance and optical characteristics, which can be suitably used in a liquid crystal display polarizing plate or an antireflection polarizing plate.

EXAMPLES

Hereinafter, the present disclosure will be described by way of the Examples, but the present disclosure is not to be limited to these Examples. It should be noted that the details of the reagents used are shown together in Table 1.

The various physical properties shown by the Examples were measured according to the following methods. Next, specific examples of the production of a fumaric acid ester monomer and a fumaric acid ester resin will be shown.

<Structural analysis of monomer precursor and monomer>

The structural analysis of the monomer precursor and the monomer was obtained by proton nuclear magnetic resonance ($^1$H-HMR) spectra analysis using a nuclear magnetic resonance measurement apparatus (NMR) (Ascend NMR 400 (Nanobay) manufactured by Bruker).

<Monomer melting point analysis>

Using differential scanning calorimetry (DSC) (Trade name: DSC250 manufactured by Shimadzu Corp.), the melting point of the monomer was determined from the endothermic peak temperature, with the temperature increase/-decrease rate of 10°C/min under a nitrogen atmosphere.

<Analysis of trans ratio in cyclohexane ring of monomer>

Using a gas chromatograph (Trade name: GC-2025 manufactured by Shimadzu Corp.), the trans ratio relative to cyclohexane ring of the monomer was obtained from the ratio of a signal derived from the trans isomer relative to the cyclohexane ring, and the signal derived from the cis isomer relative to the cyclohexane ring.

<Measurement of β relaxation temperature ($T_\beta$)>

Using differential scanning calorimetry (DSC) (Trade name: DSC7000X manufactured by Hitachi High-Tech Science Corp.), the β relaxation temperature ($T_\beta$) during second scan temperature rise was measured, by raising from 25°C to 200°C at the temperature rise rate of 10°C /min under a nitrogen atmosphere (first scan), then holding at 200°C for 10 minutes, lowering to -70°C at a cooling rate of 10°C /min, holding -70°C for 10 minutes, and raising to 200°C at the temperature rise rate of 10°C /min (second scan).

<Structural analysis of polymer (resin)>

Structural analysis of the polymer was determined by proton nuclear magnetic resonance spectroscopy ($^1$H-NMR) using a nuclear magnetic resonance analyzer(Trade name: JNM-ECZ400/L1 manufactured by JEOL Ltd.).

<Measurement of weight average molecular weight>

Using a gel permeation chromatography (GPC) apparatus (Trade name: HLC-8320GPC manufactured by Tosoh Corp.), and using two TSKgel Super HM-H columns manufactured by Tosoh Corp., the column temperature was set to 40°C, and with the tetrahydrofuran as the solvent, it was measured at 40°C to obtain the standard polystyrene equivalent value.

<Measurement of retardation characteristic (Rth)>

Using a polarization retardation measurement system (Trade name: AxoScan manufactured by Axometrics), the out-of-plane retardation Rth measured at the wavelength of 589 nm shown by formula (a) was measured.

<Thickness measurement>

Using a high-resolution linear gauge sensor (Trade name: GS-3813B manufactured by Ono Sokki Co., Ltd.), the film thickness was measured.

<Measurement of linear expansion coefficient>

A 3 cm x 3 cm film was prepared, and the film was placed on a Teflon (registered trademark) sheet. The film was placed in an oven on the Teflon (registered trademark) sheet, and temperature rise was started. On the first time, it was heated from an initial 30°C at 5°C /min, and after heating to 120°C, was held for 20 minutes. Thereafter, the temperature was cooled from 120°C at 5°C /min, cooled to 30°C, and held for 20 minutes. Similarly on the second time, it was heated from an initial 30°C at 5°C /min, and after heating to 120°C, was held for 20 minutes. Thereafter, the temperature was cooled from 120°C at 5°C /min, cooled to 30°C, and held for 20 minutes. During the above-mentioned temperature change, the temperature in the vicinity of the sample was measured with a contact-type thermocouple, and the temperature change of the film size was measured with a CCD camera. The temperature change of the film length during cooling from 120°C to 30°C on the second time was linearly approximated, and the film length change amount was calculated. From the film length change amount at this time, film temperature change amount, and the film length prior to temperature change, the linear expansion coefficient $\alpha$ represented by formula (b) was calculated. The linear expansion coefficient $\alpha$ of the film was measured as the average value of the linear expansion coefficient of the two sides of film length and width.

Synthesis Example 1

In an eggplant flask, 5.04 g of maleic anhydride (51.4 mmol) and 12.10 g (263 mmol) of ethanol were weighed, 20 mL of cyclohexane was added as a solvent, and heating and refluxing (reflux temperature: 81°C) was performed for 4 hours. The water bath was set to 70°C or higher, and unreacted ethanol and cyclohexane were distilled in an evaporator to concentrate the contents, thereby obtaining crude of the monoethyl maleate. ($^1$H-NMR (400 MHz, $CDCl_3$): $\delta$6.40 (m, 2H, CH=CH), 4.35-4.30 (q, J=8.0 Hz, 2H, O-$CH_2$), 1.37-1.33 (t, J=8.0 Hz, 3H, $CH_3$))

[Chem. 8]

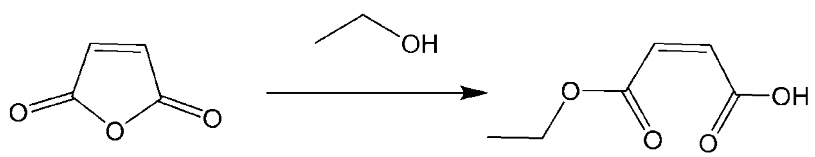

Example 1

In a flask equipped with a dropping funnel, 10.81 g (69.2 mmol) of 4-tert-butylcyclohexanol, 8.59 g (42.9 mmol) of N,N'-dicyclohexylcarbodiimide (DCC), and 0.74 g (6.06 mmol) of 4-dimethylaminopyridine (DMAP) were added, and dissolved in 20 mL of dichloromethane. Thereto, the crude obtained in Synthesis Example 1 was added dropwise and stirred in an ice bath for 2 hours. The reaction solution was filtered and the recovered solution was concentrated by an evaporator, followed by separation and purification by silica gel column chromatography (chloroform = 100 Vol%). To the obtained purified product, 20 mL of chloroform was added, then 1 mL of piperidine was added and heated and refluxed for 6 hours (reflux temperature: 61°C), thereby isomerizing fumaric acid ester (trans isomer). The reaction solution after the isomerization reaction was separated and purified by silica gel chromatography (hexane/ethyl acetate = 90/10 (Vol%)), followed by performing vacuum drying to obtain (4-tert-butylcyclohexyl)ethyl fumarate (BCEF). ($^1$H-NMR (400 MHz, $CDCl_3$): 66.83 (m, 2H, CH=CH), 4.77-4.71 (m, 1H, O-CH), 4.28-4.23 (q, J=5.3 Hz , 2H, O-$CH_2$), 2.07-0.99 (m, 9H, $CH_2$, CH), 1.33-1.30 (t, J = 5.3 Hz, 3H, $CH_3$), 0.860 (s, 9H, $(CH_3)_3$))

The melting point of the obtained BCEF was 59.4°C.

[Chem. 9]

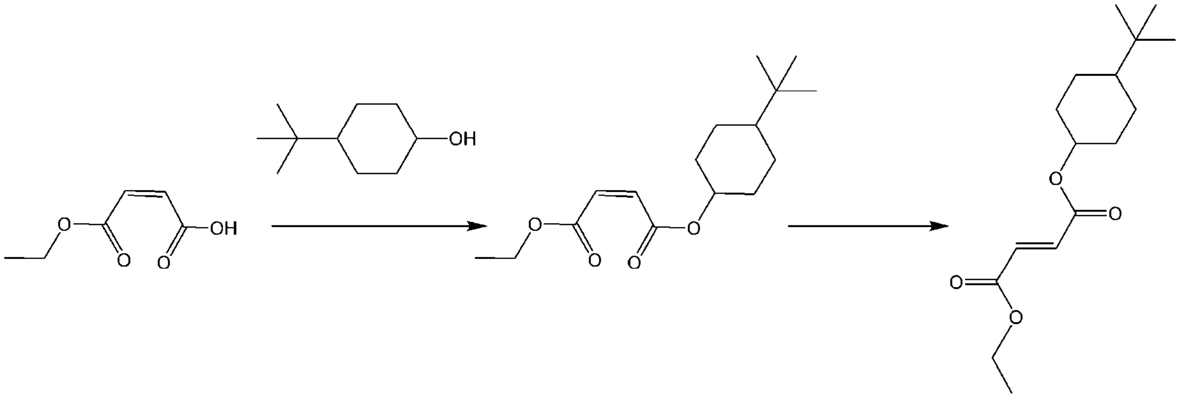

Synthesis Example 2

In an eggplant flask, 5.01 g of maleic anhydride (51.1 mmol) and 15.09 g (251 mmol) of isopropanol were weighed, 20 mL of cyclohexane was added as a solvent, and heating and refluxing (reflux temperature: 81°C) was performed for 4 hours. The water bath was set to 80°C or higher, and unreacted isopropanol and cyclohexane were distilled in an evaporator to concentrate the contents, thereby obtaining crude of the monoisopropyl maleate. ($^1$H-NMR (400 MHz, $CDCl_3$): 56.47-6.34 (m, 2H, CH=CH), 5.23-5.14 (sep, J=6.3 Hz, 1H, O-CH), 1.36-1.34 (d, J=6.3 Hz, 6H, $(CH_3)_2$))

[Chem. 10]

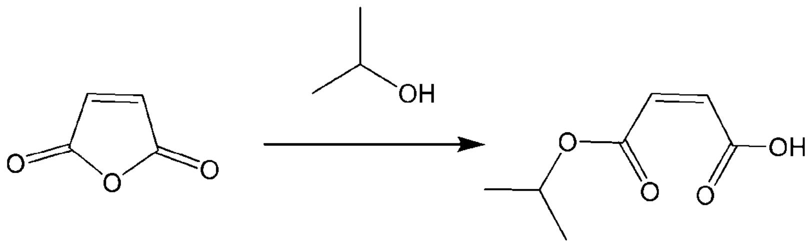

Example 2

In a flask equipped with a dropping funnel, 8.83 g (56.5 mmol) of 4-tert-butylcyclohexanol, 8.58 g (42.9 mmol) of N,N'-dicyclohexylcarbodiimide (DCC), and 0.84 g (6.86 mmol) of 4-dimethylaminopyridine (DMAP) were added, and dissolved in 20 mL of dichloromethane. Thereto, the crude obtained in Synthesis Example 2 was added dropwise, and stirred in an ice bath for 2 hours. The reaction solution was filtered and the recovered solution was concentrated by an evaporator, followed by separation and purification by silica gel column chromatography (hexane/ethyl acetate = 70/30 (Vol%)). To the obtained purified product, 20 mL of chloroform was added, then 1 mL of piperidine was added and heated and refluxed for 6 hours (reflux temperature: 61°C), thereby isomerizing. The reaction solution after the isomerization reaction was separated and purified by silica gel chromatography (hexane/ethyl acetate = 90/10 (Vol%)), followed by performing vacuum drying to obtain (4-tert-butylcyclohexyl)isopropyl fumarate (BCiPF). ($^1$H-NMR (400 MHz, $CDCl_3$): δ6.81 (m, 2H, CH=CH), 5.15-5.06 (m, 1H, O-CH), 4.78-4.70 (quintet, J=6.0 Hz, 1H, O-CH), 2.07-1.11 (m, 9H, $CH_2$, CH), 1.30-1.28 (d, J = 6.0 Hz, 6H, $(CH_3)_2$), 0.86 (s, 9H, $(CH_3)_3$))

The melting point of the obtained BCiPF was 43.8°C, and the trans ratio relative to the cyclohexane ring was 92.7%.

[Chem. 11]

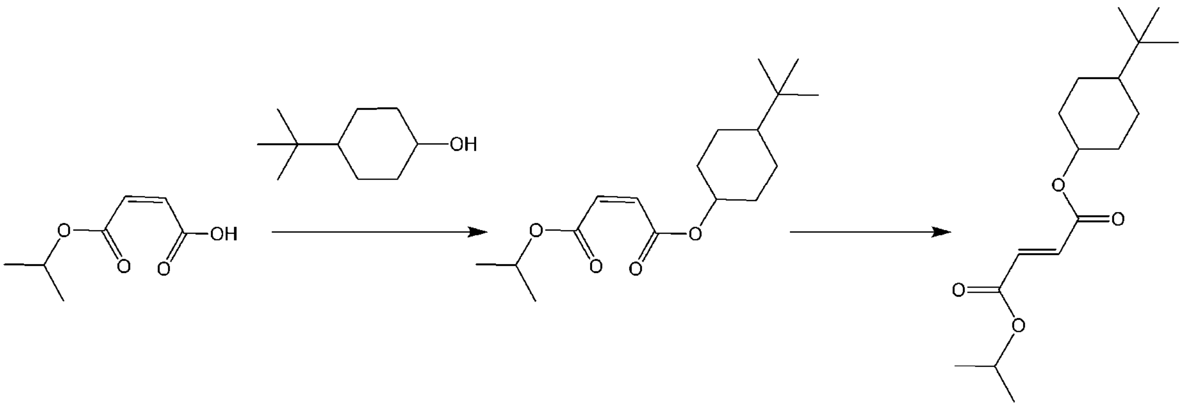

Synthesis Example 3

In an eggplant flask, 6.61 g of maleic anhydride (67.4 mmol) and 6.52 g (65.2 mmol) of cyclohexanol were weighed, 20 mL of cyclohexane was added as a solvent, and heating and refluxing (reflux temperature: 81°C) was performed for 4 hours. The water bath was set to 80°C or higher, and unreacted isopropanol and cyclohexane were distilled in an evaporator to concentrate the contents, thereby obtaining crude of the monocyclohexyl maleate. ($^1$H-NMR (400 MHz, $CDCl_3$): δ6.32-6.25 (m, 2H, CH=CH), 4.95 (quintet, J=4.0 Hz, 1H, O-CH), 1.89-1.72 (m, 10H, $CH_2$))

[Chem. 12]

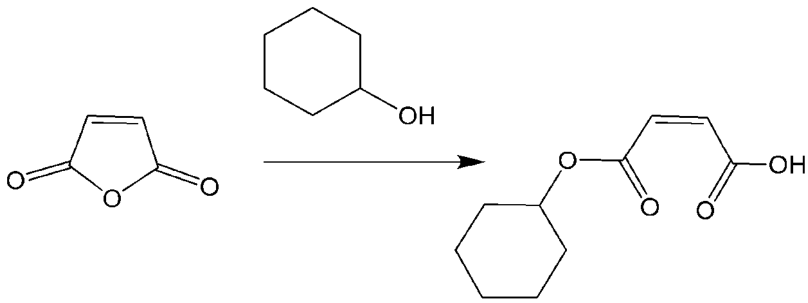

Example 3

In a flask equipped with a dropping funnel, 11.77 g (75.4 mmol) of 4-tert-butylcyclohexanol, 11.74 g (56.9 mmol) of N,N'-dicyclohexylcarbodiimide (DCC), and 0.93 g (7.6 mmol) of 4-dimethylaminopyridine (DMAP) were added, and dissolved in 20 mL of dichloromethane. Thereto, the crude obtained in Synthesis Example 3 was added dropwise and stirred in an ice bath for 2 hours. The reaction solution was filtered and the recovered solution was concentrated by an evaporator, followed by separation and purification by silica gel column chromatography (chloroform: 100 Vol%). To the obtained purified product, 20 mL of chloroform was added, then 1 mL of piperidine was added and heated and refluxed for 6 hours (reflux temperature: 61°C), thereby isomerizing. The reaction solution after the isomerization reaction was separated and purified by silica gel chromatography (hexane/ethyl acetate = 90/10 (Vol%)), followed by performing vacuum drying to obtain (4-tert-butylcyclohexyl)cyclohexyl fumarate (BCCHF). ($^1$H-NMR (400 MHz, $CDCl_3$): δ6.82 (m, 2H, CH=CH), 5.13-5.11, 4.77-4.71 (m, 1H, O-CH), 4.89-4 .86 (m, 1H, O-CH), 2.07-2.05 (m, 2H, $CH_2$), 1.85-1.01 (m, 17H, CH, $CH_2$), 0.87 (s, 9H, $(CH_3)_3$))

The melting point of the obtained BCCHF was 58.3°C.

[Chem. 13]

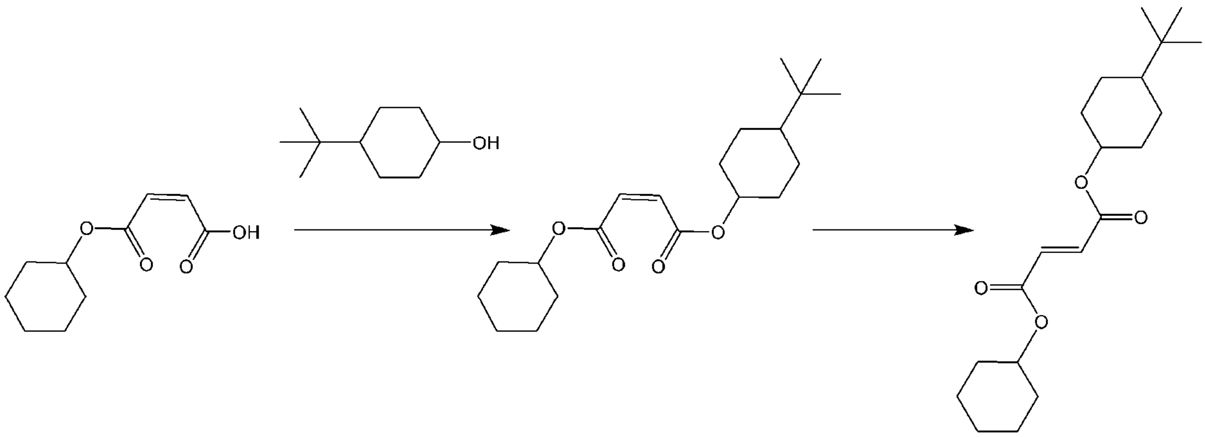

Synthesis Example 4

In a flask equipped with a dropping funnel, 9.02 g (90.0 mmol) of cyclohexanol, 6.01 g (67.4 mmol) of N,N'-dicyclohexylcarbodiimide (DCC), and 0.99 g (8.10 mmol) of 4-dimethylaminopyridine (DMAP) were added, and dissolved in 20 mL of dichloromethane. Thereto, the crude obtained in Synthesis Example 2 was added dropwise and stirred in an ice bath for 2 hours. The reaction solution was filtered and the recovered solution was concentrated by an evaporator, followed adding 20 mL of chloroform, and adding 1 mL of ethylenediamine and heating and refluxing for 4 hours (reflux temperature: 61°C), thereby isomerizing. The reaction solution after the isomerization reaction was separated and purified by silica gel chromatography (hexane/ethyl acetate = 90/10 (Vol%)), followed by performing vacuum drying to obtain cyclohexyliso-propyl fumarate (CHiPF). ($^1$H-NMR (400 MHz, $CDCl_3$): δ6.83-6.81 (m, 2H, CH=CH), 5.16-5.08 (m, 1H, O-CH), 4.90-4.84 (m, 1H, O-CH), 1.88-1.29 (m, 16H, $CH_2$, $CH_3$))

The obtained CHiPF was liquid at room temperature.

[Chem. 14]

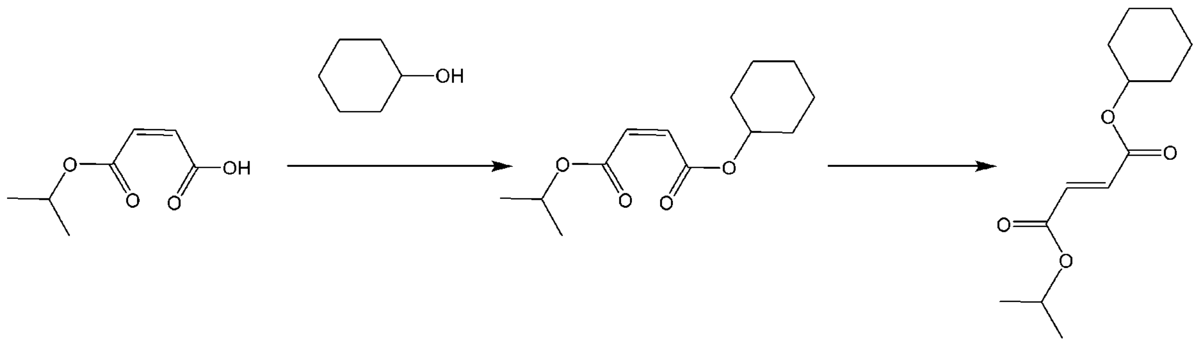

Synthesis Example 5

In an eggplant flask, 2.66 g of maleic anhydride (27.9 mmol) and 10.72 g (68.6 mmol) of 4-tert-butylcyclohexanol were weighed, 20 mL of toluene was added as a solvent, 2 mL of concentrated sulfuric acid was added as an acid catalyst, and heating refluxing (reflux temperature: 111°C) was performed for 4 hours. The water bath was set to 80°C or higher, and the toluene was distilled in an evaporator to concentrate the contents, followed by separation and purification by silica gel column chromatography (chloroform = 100 Vol%). To the purified product, 20 mL of chloroform was added, then 1 mL of piperidine was added and heated and refluxed for 4 hours (reflux temperature: 61°C), thereby isomerizing. The reaction solution after the isomerization reaction was separated and purified by silica gel chromatography (hexane/chloroform = 30/70 (Vol%)), followed by performing vacuum drying to obtain di(4-tert-butylcyclohexyl) fumarate (DBCF). ($^1$H-NMR (400 MHz, $CDCl_3$): δ6.81 (s, 2H, CH=CH), 5.13-5.11, 4.77-4.70 (m, 2H, O-CH), 2.07-1.01 (m, 18H, CH, $CH_2$), 0.87-0.86 (s, 18H, $(CH_3)_3$))

The melting point of the obtained DBCHF was 164.4°C.

[Chem. 15]

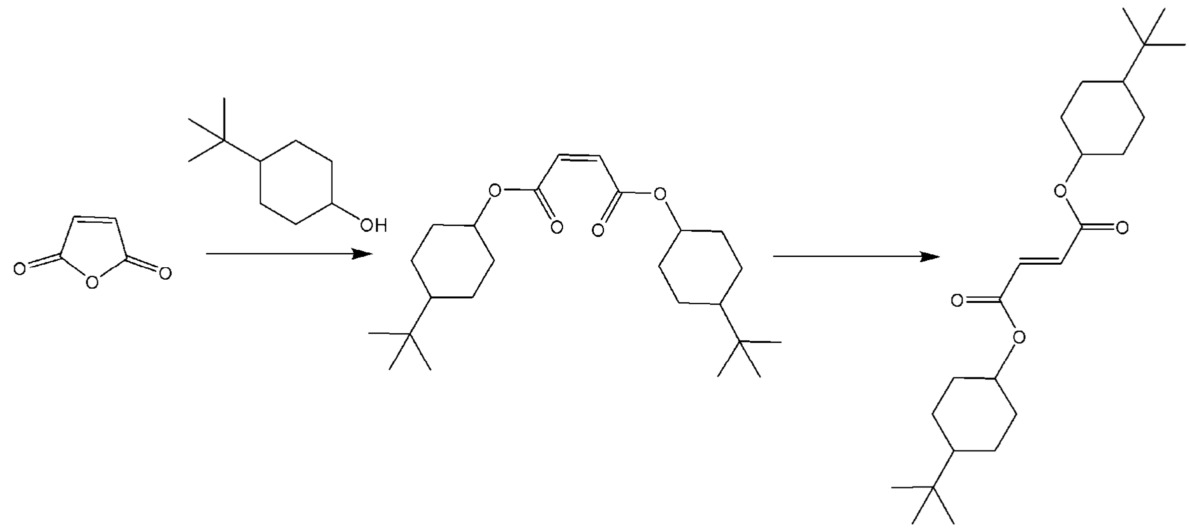

Example 4

After melting the (4-tert-butylcyclohexyl)ethyl fumarate (BCEF) obtained in Example 1 in a 70°C water bath, 1.34 g (4.74 mmol) of melted (4-tert-butylcyclohexyl)ethyl fumarate (BCEF) and 12.3 mg (0.06 mmol) of tert-butylperoxide-2-ethylhexanoate which is a polymerization initiator were placed in a 75-mL glass ampule using a glass pipette, nitrogen replacement and depressurization were repeated, followed by sealing in a reduced-pressure state. This ampule was placed in a 60°C thermostatic chamber, and radical polymerization was performed by holding for 48 hours. After completion of the polymerization reaction, 20 mL of tetrahydrofuran was added to the ampule to dissolve the polymerization product. This polymer solution was added dropwise into 100 mL of a precipitating agent (methanol/water = 75/25 (wt%)) to cause to precipitate, followed by vacuum drying for 10 hours at 80°C to obtain 0.70 g of fumaric acid ester resin (yield: 52%).

The weight average molecular weight and $T_\beta$ of the obtained fumaric acid ester resin were measured. The results thereof are shown together in Table 2. The obtained fumaric acid ester resin had a high $T_\beta$ and high weight average molecular weight.

[Chem. 16]

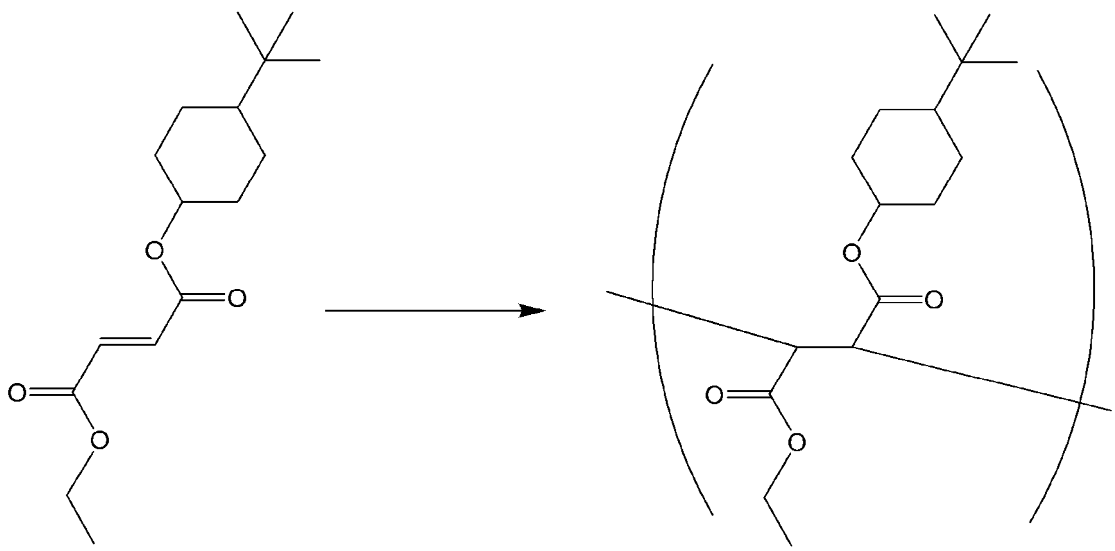

Example 5

After melting the (4-tert-butylcyclohexyl)isopropyl fumarate (BCiPF) obtained in Example 2 in a 70°C water bath, 1.93 g (6.50 mmol) of melted (4-tert-butylcyclohexyl)isopropyl fumarate (BCiPF) and 12.8 mg (0.05 mmol) of tert-butyl peroxypivalate which is a polymerization initiator were placed in a 75-mL glass ampule using a glass pipette, nitrogen replacement and depressurization were repeated, followed by sealing in a reduced-pressure state. This ampule was placed in a 45°C thermostatic chamber, and radical polymerization was performed by holding for 48 hours. After completion of the polymerization reaction, 20 mL of tetrahydrofuran was added to the ampule to dissolve the polymerization product. This polymer solution was added dropwise into 100 mL of a precipitating agent (methanol/water = 75/25 (wt%)) to cause to precipitate, followed by vacuum drying for 10 hours at 80°C to obtain 0.86 g of fumaric acid ester resin (yield: 45%).

The weight average molecular weight and $T_\beta$ of the obtained fumaric acid ester resin were measured. The results thereof are shown together in Table 2. The obtained fumaric acid ester resin had a high $T_\beta$ and high weight average molecular weight.

[Chem. 17]

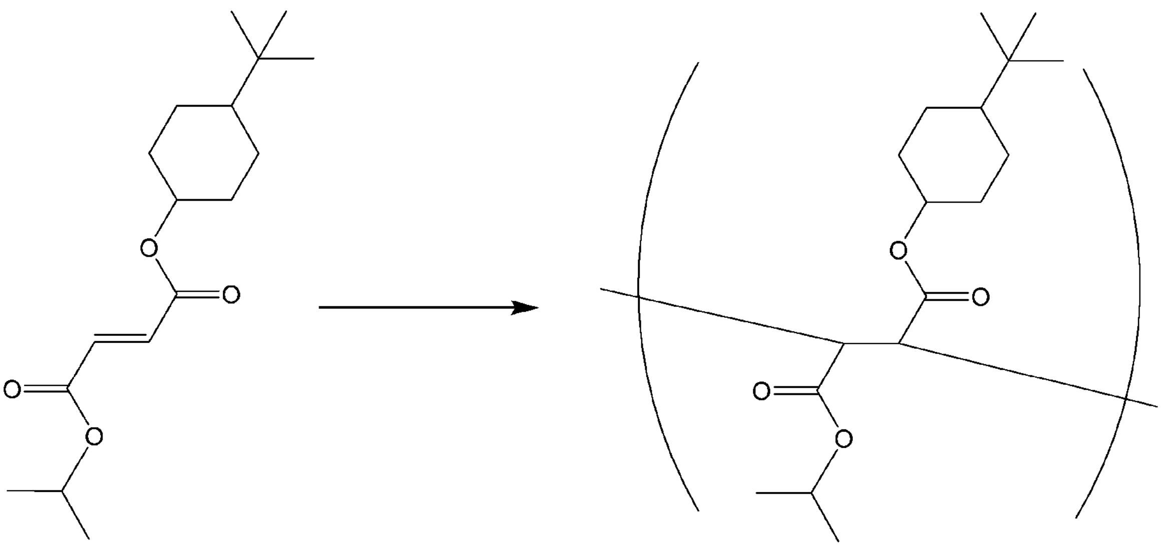

Example 6

After melting the (4-tert-butylcyclohexyl)cyclohexyl fumarate (BCCHF) obtained in Example 3 in a 70°C water bath, 1.07 g (3.17 mmol) of melted (4-tert-butylcyclohexyl)cyclohexyl fumarate (BCCHF) and 6.0 mg (0.03 mmol) of tert-butylperoxide-2-ethylhexanoate which is a polymerization initiator were placed in a 75-mL glass ampule using a glass pipette, nitrogen replacement and depressurization were repeated, followed by sealing in a reduced-pressure state. This ampule was placed in a 60°C thermostatic chamber, and radical polymerization was performed by holding for 48 hours. After completion of the polymerization reaction, 10 mL of tetrahydrofuran was added to the ampule to dissolve the polymerization product. This polymer solution was added dropwise into 50 mL of a precipitating agent (methanol/water = 75/25 (wt%)) to cause to precipitate, the obtained precipitate was washed with 30 mL of methanol, followed by vacuum drying for 10 hours at 80°C to obtain 0.31 g of fumaric acid ester resin (yield: 29%).

The weight average molecular weight and $T_\beta$ of the obtained fumaric acid ester resin were measured. The results thereof are shown together in Table 2. The obtained fumaric acid ester resin had a high $T_\beta$ and high weight average molecular weight.

[Chem. 18]

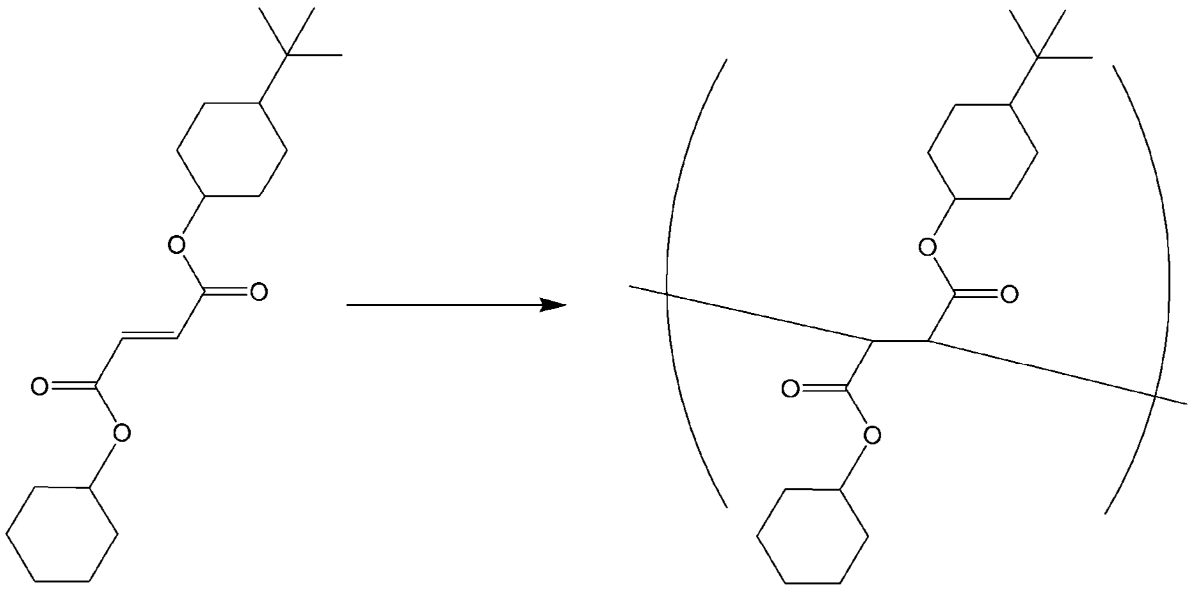

Example 7

After melting the (4-tert-butylcyclohexyl)ethyl fumarate (BCEF) obtained in Example 1 in a 70°C water bath, 0.76 g (2.69 mmol) of melted (4-tert-butylcyclohexyl)ethyl fumarate (BCEF) was placed in a 75-mL glass ampule using a glass pipette. To a 6-mL glass sample bottle, 0.32 g (1.59 mmol) of diisopropyl fumarate (DiPF) and 22.7 mg (0.10 mmol) of tert-butylperoxide-2-ethylhexanoate which is a polymerization initiator were placed, and the initiator DiPF solution was prepared. To the glass ampule in which BCEF was placed, 0.34 g (DiPF (0.30 mmol) of the prepared initiator DiPF solution and tert-butylperoxide-2-ethylhexanoate (0.02 mmol)) were placed, and nitrogen replacement and depressurization were repeated, followed by sealing in a reduced-pressure state. This ampule was placed in a 60°C thermostatic chamber, and radical polymerization was performed by holding for 96 hours. After completion of the polymerization reaction, 10 mL of tetrahydrofuran was added to the ampule to dissolve the polymerization product. This polymer solution was added dropwise into 50 mL of a precipitating agent (methanol/water = 75/25 (wt%)) to cause to precipitate, followed by vacuum drying for 10 hours at 80°C to obtain 0.31 g of fumaric acid ester resin (yield: 38%).

The resin composition, weight average molecular weight, and $T_\beta$ of the obtained fumaric acid ester resin were measured. The results thereof are shown together in Table 2. The obtained fumaric acid ester resin had a high $T_\beta$ and high weight average molecular weight.

[Chem. 19]

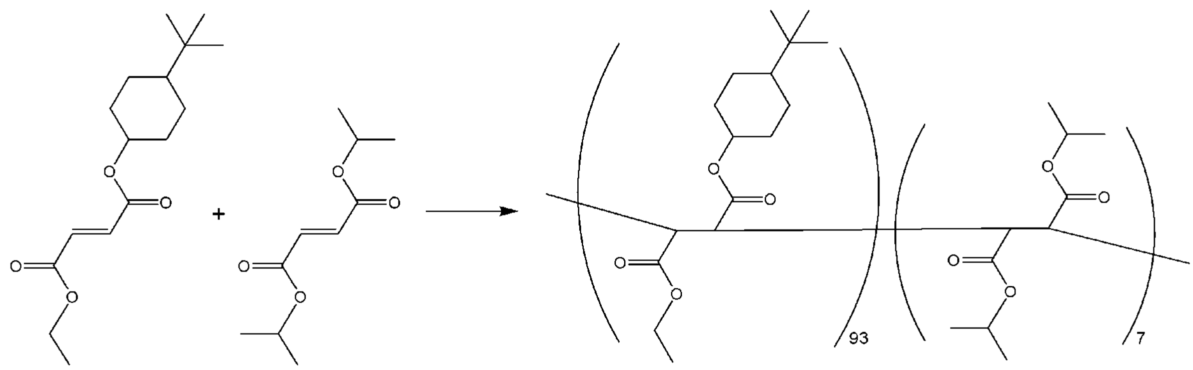

Example 8

To a 6-mL glass sample bottle, 1.18 g (4.32 mmol) of the (4-tert-butylcyclohexyl)isopropyl fumarate (BCiPF) obtained in Example 2 and 0.20 g (1.11 mmol) of diisopropyl fumarate (DiPF) were placed, and melted in a 70°C water bath to prepare a monomer solution. Using a glass pipette, 1.38 g (BCiPF (3.97 mmol) of monomer solution, DiPF (1.02 mmol))

and 10.4 mg (0.04 mmol) of tert-butyl peroxypivalate which is a polymerization initiator were placed in a 75-mL glass ampule, nitrogen replacement and depressurization were repeated, followed by sealing in a reduced-pressure state. This ampule was placed in a 45°C thermostatic chamber, and radical polymerization was performed by holding for 48 hours. After completion of the polymerization reaction, 20 mL of tetrahydrofuran was added to the ampule to dissolve the polymerization product. This polymer solution was added dropwise into 100 mL of a precipitating agent (methanol/water = 75/25 (wt%)) to cause to precipitate, followed by vacuum drying for 10 hours at 80°C to obtain 0.90 g of fumaric acid ester resin (yield: 65%).

The resin composition, weight average molecular weight, and $T_\beta$ of the obtained fumaric acid ester resin were measured. The results thereof are shown together in Table 2. The obtained fumaric acid ester resin had a high $T_\beta$ and high weight average molecular weight.

[Chem. 20]

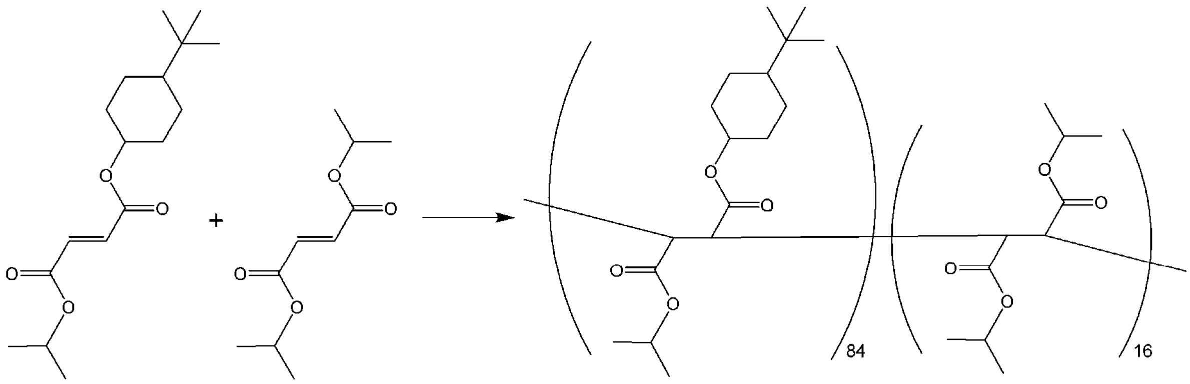

Example 9

To a 6-mL glass sample bottle, 0.67 g (2.27 mmol) of the (4-tert-butylcyclohexyl)isopropyl fumarate (BCiPF) obtained in Example 2 and 0.30 g (1.51 mmol) of diisopropyl fumarate (DiPF) were placed, and melted in a 70°C water bath to prepare a monomer solution. Using a glass pipette, 0.89 g of monomer solution (BCiPF (2.07 mmol), DiPF (1.37 mmol)) and 8.20 mg (0.03 mmol) of tert-butyl peroxypivalate which is a polymerization initiator were placed in a 75-mL glass ampule, nitrogen replacement and depressurization were repeated, followed by sealing in a reduced-pressure state. This ampule was placed in a 45°C thermostatic chamber, and radical polymerization was performed by holding for 48 hours. After completion of the polymerization reaction, 10 mL of tetrahydrofuran was added to the ampule to dissolve the polymerization product. This polymer solution was added dropwise into 50 mL of a precipitating agent (methanol/water = 75/25 (wt%)) to cause to precipitate, the obtained precipitate was washed with 50 mL of methanol, followed by vacuum drying for 10 hours at 80°C to obtain 0.56 g of fumaric acid ester resin (yield: 63%).

The resin composition, weight average molecular weight, and $T_\beta$ of the obtained fumaric acid ester resin were measured. The results thereof are shown together in Table 2. The obtained fumaric acid ester resin had a high $T_\beta$ and high weight average molecular weight.

[Chem. 21]

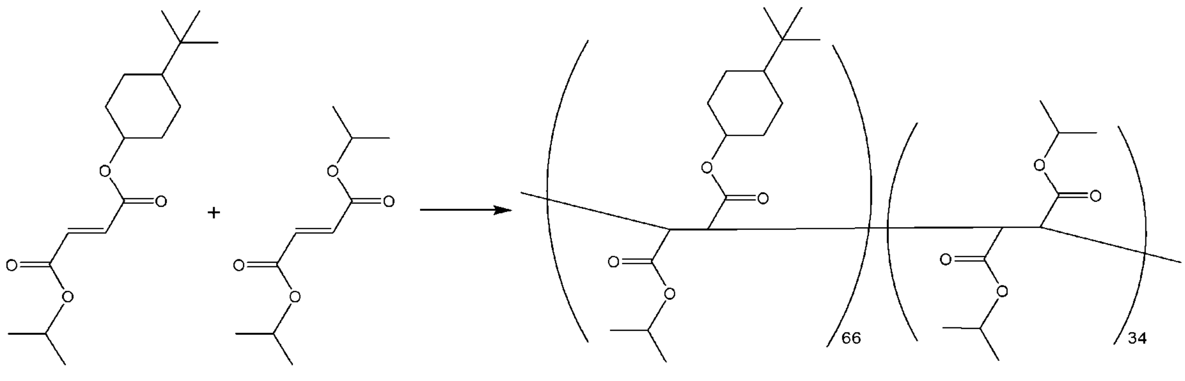

Comparative Example 1

In a 75-mL glass ampule, 13.01 g (65.0 mmol) of diisopropyl fumarate and 136.0 mg (0.55 mmol) of tert-butyl peroxypivalate which is a polymerization initiator were placed, nitrogen replacement and depressurization were repeated, followed by sealing in a reduced-pressure state. This ampule was placed in a 50°C thermostatic chamber, and radical polymerization was performed by holding for 24 hours. After completion of the polymerization reaction, the polymerization product was retrieved from the ampule, and 130 mL of tetrahydrofuran was added thereto to dissolve the polymerization product. This polymer solution was added dropwise into 650 mL methanol to cause to precipitate, followed by vacuum drying for 10 hours at 80°C to obtain 11.28 g of fumaric acid ester resin (yield: 43%).

The weight average molecular weight and $T_\beta$ of the obtained fumaric acid ester resin were measured. The results thereof are shown together in Table 2. The obtained fumaric acid ester resin was a resin having high weight average molecular weight, but low $T_\beta$. The resin obtained from the fumaric acid ester monomer without a rigid structure was confirmed to have lowered $T_\beta$.

[Chem. 22]

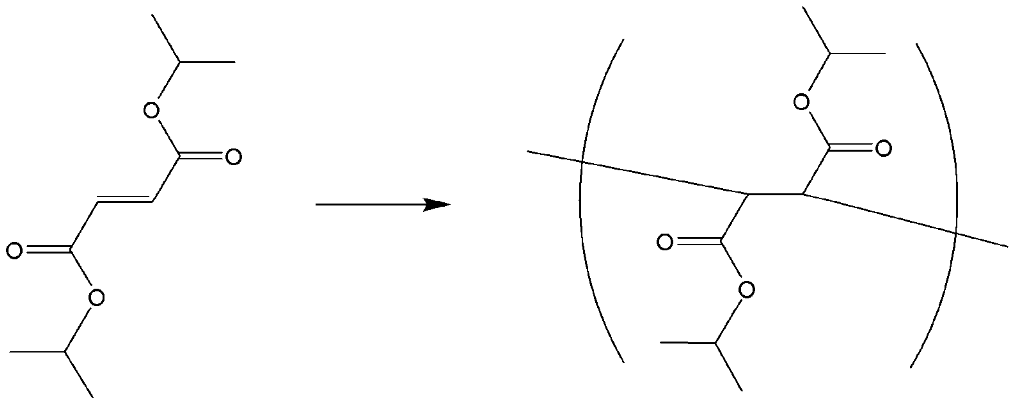

Comparative Example 2

To a 6-mL glass sample bottle, 2.01 g (8.35 mmol) of the cyclohexylisopropyl fumarate (CHiPF) obtained in Synthesis Example 4 and 16.6 g (0.07 mmol) of tert-butyl peroxypivalate were placed to prepare a polymer solution. Using a glass pipette, 1.94 g of polymer solution (CHiPF (8.00 mmol) and tert-butyl peroxypivalate (0.06 mmol)) were placed in a 75-mL glass ampule, nitrogen replacement and depressurization were repeated, followed by sealing in a reduced-pressure state. This ampule was placed in a 45°C thermostatic chamber, and radical polymerization was performed by holding for 48 hours. After completion of the polymerization reaction, 20 mL of tetrahydrofuran was added to the ampule to dissolve the polymerization product. This polymer solution was added dropwise into 100 mL of a precipitating agent (methanol/water = 75/25 (wt%)) to cause to precipitate, followed by vacuum drying for 10 hours at 80°C to obtain 1.10 g of fumaric acid ester resin (yield: 57%).

The weight average molecular weight, and $T_\beta$ of the obtained fumaric acid ester resin were measured. The results thereof are shown together in Table 2. The obtained fumaric acid ester resin was a resin having high weight average molecular weight, but low $T_\beta$. The resin obtained from the fumaric acid ester monomer having an unsubstituted cyclohexyl ring at one end was confirmed to have lowered $T_\beta$.

[Chem. 23]

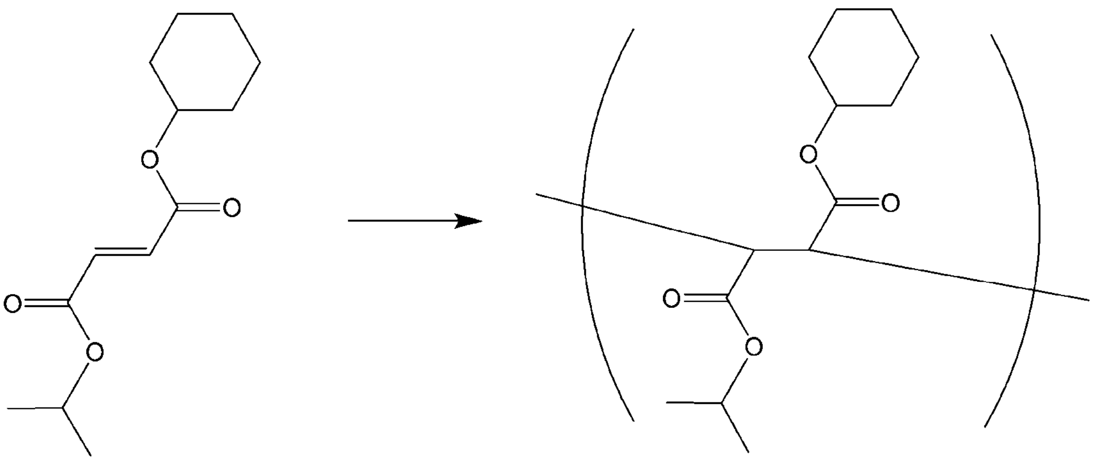

Comparative Example 3

To a 15-mL polymerization tube with ground glass joint, 0.988 g (2.51 mmol) of the di(4-tert-butylcyclohexyl) fumarate (DBCF) obtained in Synthesis Example 5 and 2.6 mg (0.013 mmol) of tert-butyl perhydroxyoxide were placed, and after repeating nitrogen replacement and depressurization, a nitrogen environment was established. This polymerization tube with ground glass joint was placed in a thermostatic chamber at 172°C, and radical polymerization was performed by holding for 6 hours. After polymerization reaction completion, 5 mL of chloroform was added to the polymerization tube with slide, and the polymerization product was dissolved. This polymer solution was added dropwise into 200 mL of ethanol warmed to 60°C to cause to precipitate, followed by vacuum drying for 3 hours at 80°C to obtain 0.322 g of fumaric acid ester resin (yield: 32.6%).

The weight average molecular weight, and $T_{\beta}$ of the obtained fumaric acid ester resin were measured. The results thereof are shown together in Table 2. The obtained fumaric acid ester resin was a resin having high $T_{\beta}$, but was low weight average molecular weight. The fumaric acid ester monomer having a rigid substituent at both ends was confirmed to make a resin having high melting point, and thus having higher polymerization temperature for melting the monomer, resulting in low weight average molecular weight.

[Chem. 24]

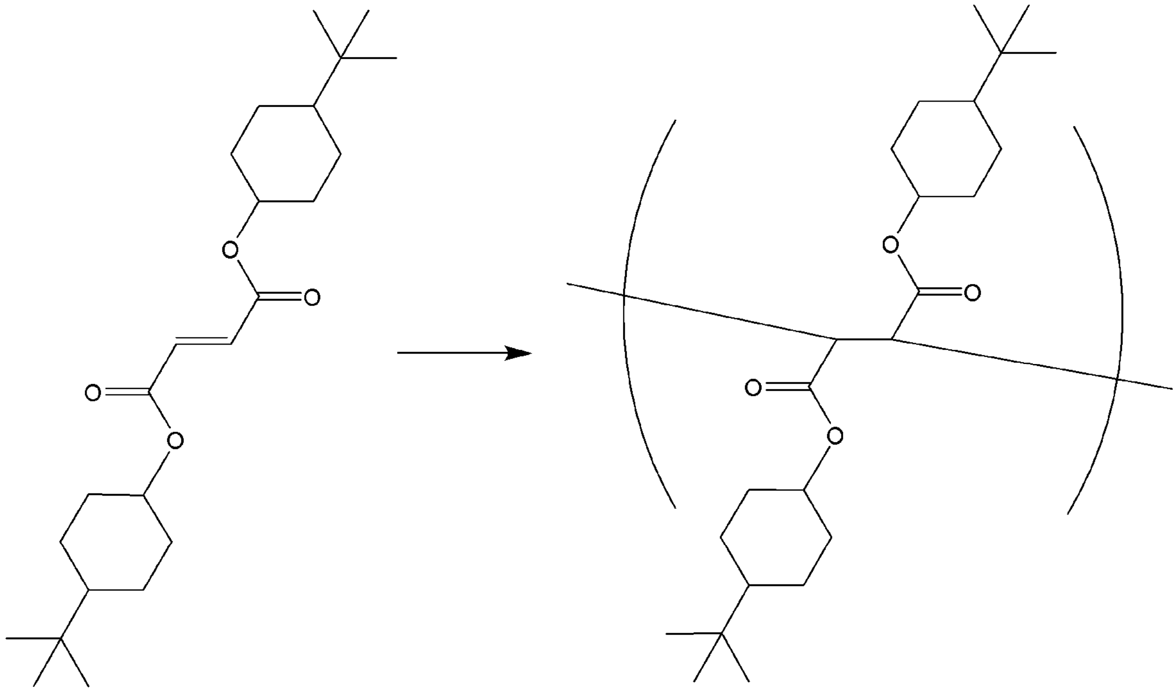

Example 10

In a tetrahydrofuran solution, 0.4 g of the fumaric acid ester resin obtained by Example 5 was dissolved to make a 10 wt% resin solution. This solution was cast onto a polyethylene terephthalate substrate (PET100-TP03 manufactured by PANAC) by a coater, and dried in two stages at drying temperatures of 50°C and 130°C to prepare a film with 19 μm film thickness. The prepared fumaric acid ester resin film was peeled from the substrate, and the linear expansion coefficient and retardation characteristic of the film alone were evaluated. The results thereof are shown together in Table 3. The obtained film was superior in viewing angle characteristic, and dimensional stability while high temperature, and thus was favorable as an optical film.

Example 11

In a tetrahydrofuran solution, 0.4 g of the fumaric acid ester resin obtained by Example 9 was dissolved to make a 11 wt% resin solution. This solution was cast onto a polyethylene terephthalate substrate (PET100-TP03 manufactured by PANAC) by a coater, and dried in two stages at drying temperatures of 50°C and 130°C to prepare a film with 20 μm film thickness. The prepared fumaric acid ester resin film was peeled from the substrate, and the linear expansion coefficient and retardation characteristic of the film alone were evaluated. The results thereof are shown together in Table 3. The obtained film was superior in viewing angle characteristic, and dimensional stability during high temperature, and thus was favorable as an optical film.

Comparative Example 4

In a tetrahydrofuran solution, 0.4 g of the fumaric acid ester resin obtained by Comparative Example 1 was dissolved to make a 15 wt% resin solution. This solution was cast onto a polyethylene terephthalate substrate (PET100-TP03 manufactured by PANAC) by a coater, and dried in two stages at drying temperatures of 50°C and 130°C to prepare a film with 21 μm film thickness. The prepared fumaric acid ester resin film was peeled from the substrate, and the linear expansion coefficient and retardation characteristic of the film alone were evaluated. The results thereof are shown together in Table 3. The obtained film had a large coefficient of linear expansion due to the low $T_\beta$ of the fumaric acid ester resin, and did not have the desired heat resistance.

Comparative Example 5

In a tetrahydrofuran solution, 0.4 g of the fumaric acid ester resin obtained by Comparative Example 2 was dissolved to make a 15 wt% resin solution. This solution was cast onto a polyethylene terephthalate substrate (PET100-TP03 manufactured by PANAC) by a coater, and dried in two stages at drying temperatures of 50°C and 130°C to prepare a

film with 19 μm film thickness. The prepared fumaric acid ester resin film was peeled from the substrate, and the linear expansion coefficient and retardation characteristic of the film alone were evaluated. The results thereof are shown together in Table 3. The obtained film had a large coefficient of linear expansion due to the low $T_\beta$ of the fumaric acid ester resin, and did not have the desired heat resistance.

Comparative Example 6

In a tetrahydrofuran solution, 0.4 g of the fumaric acid ester resin obtained by Comparative Example 3 was dissolved to make a 30 wt% resin solution. This solution was cast onto a polyethylene terephthalate substrate (PET100-TP03 manufactured by PANAC) by a coater, and dried in two stages at drying temperatures of 50°C and 130°C to prepare a film with 20 μm film thickness. Although it was attempted to peel the prepared fumaric acid ester resin film from the substrate, cracks occurred throughout the surface. Since the weight average molecular weight of the fumaric acid ester resin was low, and strength insufficient, it was unsuitable as an optical film.

Synthesis Example 6

In an eggplant flask, 10.2 g of maleic anhydride (103.98 mmol) and 13.01 g (216.47 mmol) of isopropanol were weighed, 20 mL of cyclohexane was added as a solvent, and heating and refluxing (reflux temperature: 81°C) was performed for 4 hours. The water bath was set to 80°C or higher, and unreacted isopropanol and cyclohexane were distilled in an evaporator to concentrate the contents, thereby obtaining crude of the monoisopropyl maleate. ($^1$H-NMR (400 MHz, $CDCl_3$): 56.47-6.34 (m, 2H, CH=CH), 5.23-5.14 (sep, J=6.3 Hz, 1H, O-CH), 1.36-1.34 (d, J=6.3 Hz, 6H, $(CH_3)_2$))

[Chem. 25]

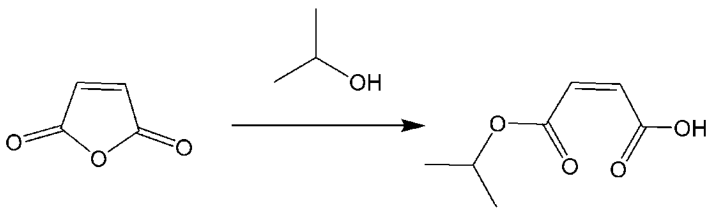

Synthesis Example 7

In an eggplant flask, 5.01 g of maleic anhydride (51.1 mmol) and 15.09 g (251 mmol) of isopropanol were weighed, 20 mL of cyclohexane was added as a solvent, and heating and refluxing (reflux temperature: 81°C) was performed for 4 hours. The water bath was set to 80°C or higher, and unreacted isopropanol and cyclohexane were distilled in an evaporator to concentrate the contents, thereby obtaining crude of the monoisopropyl maleate. ($^1$H-NMR (400 MHz, $CDCl_3$): 66.47-6.34 (m, 2H, CH=CH), 5.23-5.14 (sep, J=6.3 Hz, 1H, O-CH), 1.36-1.34 (d, J=6.3 Hz, 6H, $(CH_3)_2$))

[Chem. 26]

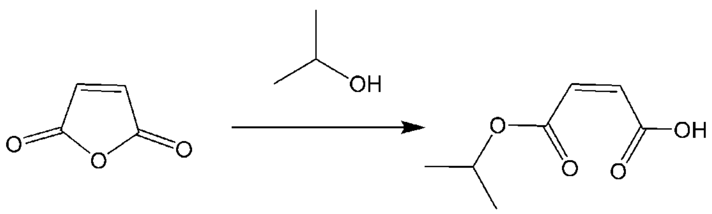

Example 12

In a flask equipped with a dropping funnel, 18.57 g (118.83 mmol) of 4-tert-butylcyclohexanol (cis/trans mixture), 17.16 g (110.53 mmol) of 1-(3-dimethylaminopropyl)-3-ethylcarbodiimide (EDC), and 1.72 g (14.08 mmol) of 4-dimethylaminopyridine (DMAP) were added, and dissolved in 20 mL of dichloromethane. Thereto, the crude obtained in Synthesis Example 6 was added dropwise and stirred in an ice bath for 2 hours. The reaction solution was liquid separation refined using a saturated saline solution, and the recovered organic phase was concentrated by an evaporator, followed by drying

with anhydrous sodium sulfate. Separation and purification was performed on the dried organic phase by silica gel chromatography (chloroform = 100 Vol%). To the obtained purified product, 20 mL of chloroform was added, then 1 mL of piperidine was added and heated and refluxed for 4 hours (reflux temperature: 61°C), thereby isomerizing. The reaction solution after the isomerization reaction was separated and purified by silica gel chromatography (hexane/ethyl acetate = 80/20 (Vol%)), followed by performing vacuum drying to obtain (4-tert-butylcyclohexyl)isopropyl fumarate (BCiPF). ($^1$H-NMR (400 MHz, $CDCl_3$): δ6.81 (m, 2H, CH=CH), 5.15-5.06 (m, 1H, O-CH), 4.78-4.70 (quintet, J=6.0 Hz, 1H, O-CH), 2.07-1.11 (m, 9H, $CH_2$, CH), 1.30-1.28 (d, J = 6.0 Hz, 6H, $(CH_3)_2$), 0.86 (s, 9H, $(CH_3)_3$))

The melting point of the obtained BCiPF was 43.8°C, and the trans ratio relative to the cyclohexane ring was 90.8%.

[Chem. 27]

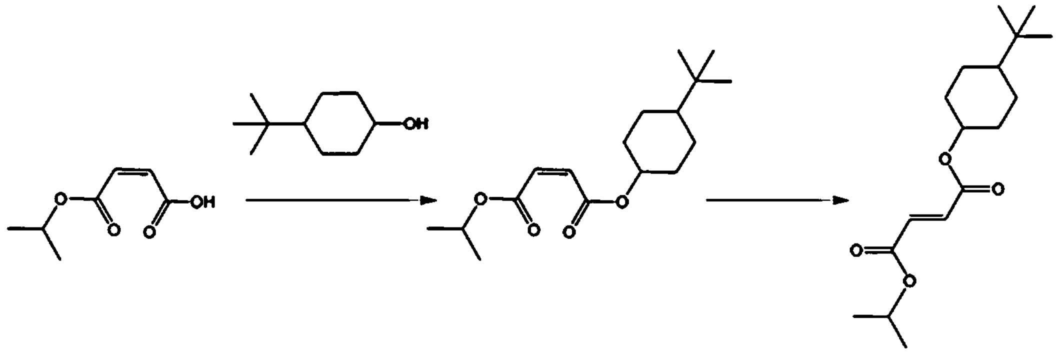

Example 13

In a flask equipped with a dropping funnel, 8.83 g (36.5 mmol) of 4-tert-butylcyclohexanol (cis/trans mixture), 8.58 g (42.9 mmol) of N,N-dicyclohexylcarbodiimide (DDC), and 0.84 g (6.86 mmol) of 4-dimethylaminopyridine (DMAP) were added, and dissolved in 20 mL of dichloromethane. Thereto, the crude obtained in Synthesis Example 7 was added dropwise and stirred in an ice bath for 2 hours. The reaction solution was liquid separation refined using a saturated saline solution, and the recovered organic phase was concentrated by an evaporator, followed by drying with anhydrous sodium sulfate. Separation and purification was performed on the dried organic phase by silica gel chromatography (hexane:ethyl acetate = 70:30 Vol%). To the obtained purified product, 20 mL of chloroform was added, then 0.5 mL of piperidine was added and heated and refluxed for 6 hours (reflux temperature: 61°C), thereby isomerizing. The reaction solution after the isomerization reaction was separated and purified by silica gel chromatography (hexane/ethyl acetate = 80/20 (Vol%)), followed by performing vacuum drying to obtain (4-tert-butylcyclohexyl)isopropyl fumarate (BCiPF). ($^1$H-NMR (400 MHz, $CDCl_3$): δ6.81 (m, 2H, CH=CH), 5.15-5.06 (m, 1H, O-CH), 4.78-4.70 (quintet, J=6.0 Hz, 1H, O-CH), 2.07-1.11 (m, 9H, CH2, CH), 1.30-1.28 (d, J = 6.0 Hz, 6H, $(CH_3)_2$), 0.86 (s, 9H, $(CH_3)_3$))

The melting point of the obtained BCiPF was 44°C, and the trans ratio relative to the cyclohexane ring was 90.3%.

[Chem. 28]

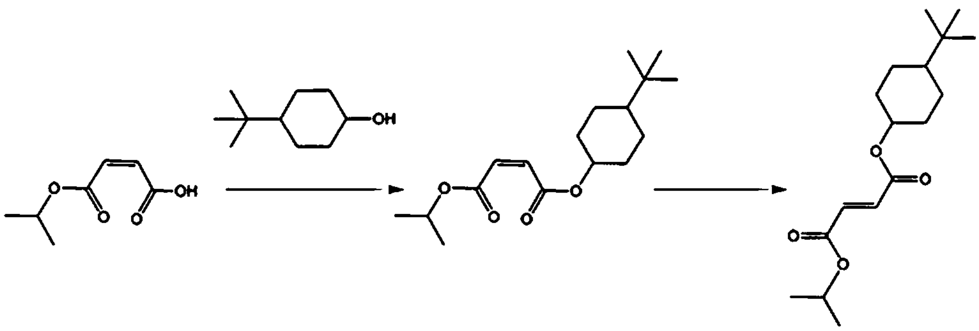

Example 14

In a flask equipped with a dropping funnel, 5.08 g (32.51 mmol) of 4-tert-butylcyclohexanol (cis isomer), 5.33 g (25.83 mmol) of N,N-dicyclohexylcarbodiimide (DDC), and 0.43 g (3.52 mmol) of 4-dimethylaminopyridine (DMAP) were added, and dissolved in 20 mL of dichloromethane. Thereto, the crude obtained in Synthesis Example 6 was added dropwise and stirred in an ice bath for 2 hours. The reaction solution was filtered and the recovered solution was concentrated by an evaporator, followed by separation and purification by silica gel column chromatography (chloroform = 100 Vol%). To the obtained purified product, 20 mL of chloroform was added, then 1 mL of piperidine was added and heated and refluxed for 4 hours (reflux temperature: 61°C), thereby isomerizing. The reaction solution after the isomerization reaction was separated and purified by silica gel chromatography (hexane/ethyl acetate = 90/10 (Vol%)), followed by performing vacuum drying to obtain (4-tert-butylcyclohexyl)isopropyl fumarate (BCiPF) of the cis isomer. ($^1$H-NMR (400 MHz, $CDCl_3$): δ6.83 (dd', 2H, CH=CH), 5.15-5.06 (m, 1H, O-CH), 4.78-4.70 (quintet, J=6. 0 Hz, 1H, O-CH), 2.07-1.11 (m, 9H, $CH_2$, CH), 1.30-1.28 (d, J = 6.0 Hz, 6H, $(CH_3)_2$), 0.86 (s, 9H, $(CH_3)_3$))

The obtained BCiPF was a liquid at room temperature, and the trans ratio relative to the cyclohexane ring was 0.4%.

[Chem. 29]

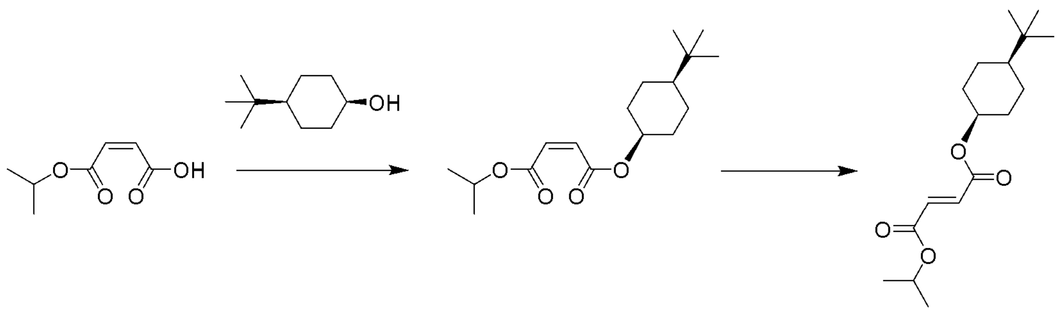

Example 15

To a 6-mL glass sample bottle, 0.60 g (2.01 mmol) of the (4-tert-butylcyclohexyl)isopropyl fumarate (BCiPF) obtained in Example 12 and 0.61 g (3.02 mmol) of diisopropyl fumarate (DiPF) were placed, and melted in a 70°C water bath to prepare a monomer solution. Using a glass pipette, 1.14 g of monomer solution (BCiPF (1.91 mmol), DiPF (2.87 mmol)) and 11.2 mg (0.04 mmol) of tert-butyl peroxypivalate which is a polymerization initiator were placed in a 75-mL glass ampule, nitrogen replacement and depressurization were repeated, followed by sealing in a reduced-pressure state. This ampule was placed in a 45°C thermostatic chamber, and radical polymerization was performed by holding for 48 hours. After completion of the polymerization reaction, 20 mL of tetrahydrofuran was added to the ampule to dissolve the polymerization product. This polymer solution was added dropwise into 100 mL of a precipitating agent (methanol/water = 75/25 (wt%)) to cause to precipitate, the obtained precipitate was washed with 50 mL of methanol, followed by vacuum drying for 10 hours at 80°C to obtain 0.87 g of fumaric acid ester resin (yield: 76%).

The resin composition, weight average molecular weight, and $T_\beta$ of the obtained fumaric acid ester resin were measured. The results thereof are shown together in Table 2. The obtained fumaric acid ester resin had a high $T_\beta$ and high weight average molecular weight.

[Chem. 30]

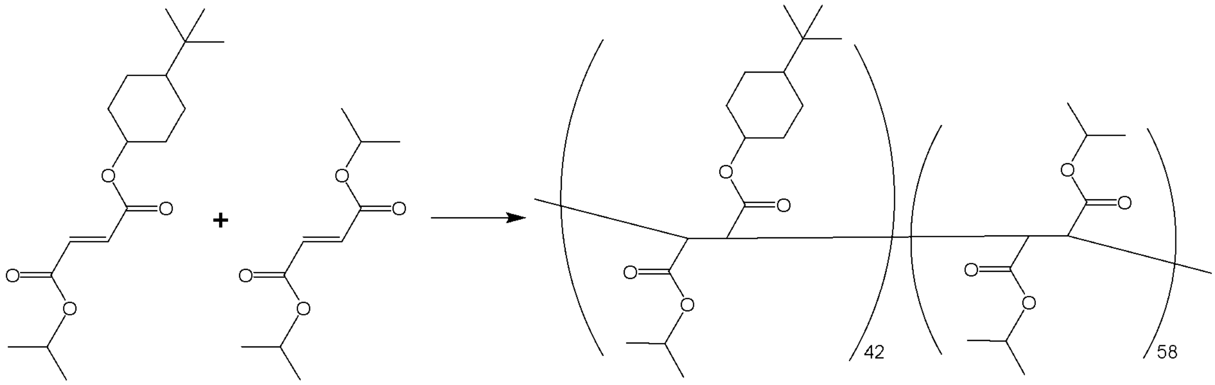

Example 16

To a 6-mL glass sample bottle, 1.60 g (5.4 mmol) of the (4-tert-butylcyclohexyl)isopropyl fumarate (BCiPF) obtained in Example 12 were placed, and melted in a 70°C water bath to prepare a monomer solution. Using a glass pipette, 1.47 g of monomer solution (BCiPF (5.0 mmol)), and 12.0 mg (0.05 mmol) of tert-butyl peroxypivalate which is a polymerization initiator were placed in a 75-mL glass ampule, nitrogen replacement and depressurization were repeated, followed by sealing in a reduced-pressure state. This ampule was placed in a 50°C thermostatic chamber, and radical polymerization was performed by holding for 24 hours. After completion of the polymerization reaction, 20 mL of tetrahydrofuran was added to the ampule to dissolve the polymerization product. This polymer solution was added dropwise into 100 mL of a precipitating agent (methanol/water = 75/25 (wt%)) to cause to precipitate, the obtained precipitate was washed with 50 mL of methanol, followed by vacuum drying for 10 hours at 80°C to obtain 1.00 g of fumaric acid ester resin (yield: 68%).

The resin composition, weight average molecular weight, and $T_\beta$ of the obtained fumaric acid ester resin were measured. The results thereof are shown together in Table 2. The obtained fumaric acid ester resin had a high $T_\beta$ and high weight average molecular weight.

[Chem. 31]

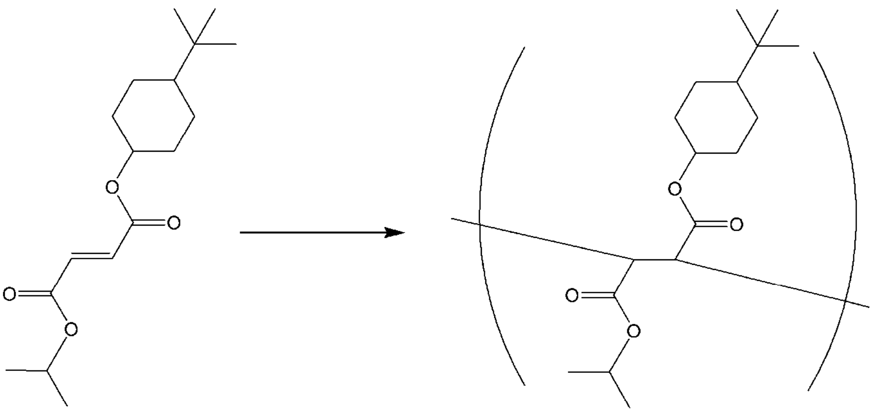

Example 17

To a 6-mL glass sample bottle, 1.35 g (4.6 mmol) of the (4-tert-butylcyclohexyl)isopropyl fumarate (BCiPF) obtained in Example 13 and 0.13 g (0.7 mmol) of diethyl fumarate (DEF) were placed, and melted in a 70°C water bath to prepare a monomer solution. Using a glass pipette, 1.37 g of monomer solution (BCiPF (4.3 mmol), DEF (0.65 mmol)) and 10.2 mg (0.04 mmol) of tert-butyl peroxypivalate which is a polymerization initiator were placed in a 75-mL glass ampule, nitrogen replacement and depressurization were repeated, followed by sealing in a reduced-pressure state. This ampule was placed in a 50°C thermostatic chamber, and radical polymerization was performed by holding for 24 hours. After completion of the polymerization reaction, 20 mL of tetrahydrofuran was added to the ampule to dissolve the polymerization product. This polymer solution was added dropwise into 100 mL of a precipitating agent (methanol/water = 75/25 (wt%)) to cause to precipitate, the obtained precipitate was washed with 50 mL of methanol, followed by vacuum drying for 10 hours at 80°C to obtain 0.57 g of fumaric acid ester resin (yield: 41%).

The resin composition, weight average molecular weight, and $T_\beta$ of the obtained fumaric acid ester resin were measured. The results thereof are shown together in Table 2. The obtained fumaric acid ester resin had a high $T_\beta$ and high weight average molecular weight.

[Chem. 32]

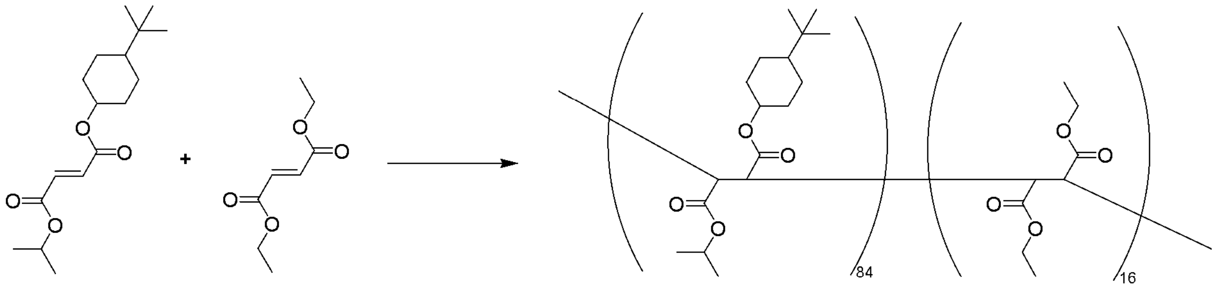

Example 18

To a 6-mL glass sample bottle, 1.52 g (5.1 mmol) of the (4-tert-butylcyclohexyl)isopropyl fumarate (BCiPF) obtained in Example 14 were placed, and melted in a 70°C water bath to prepare a monomer solution. Using a glass pipette, 1.42 g of monomer solution (BCiPF (4.8 mmol)), and 8.8 mg (0.04 mmol) of tert-butyl peroxypivalate which is a polymerization initiator were placed in a 75-mL glass ampule, nitrogen replacement and depressurization were repeated, followed by sealing in a reduced-pressure state. This ampule was placed in a 50°C thermostatic chamber, and radical polymerization was performed by holding for 24 hours. After completion of the polymerization reaction, 20 mL of tetrahydrofuran was added to the ampule to dissolve the polymerization product. This polymer solution was added dropwise into 100 mL of a precipitating agent (methanol/water = 75/25 (wt%)) to cause to precipitate, the obtained precipitate was washed with 50 mL of methanol, followed by vacuum drying for 10 hours at 80°C to obtain 0.26 g of fumaric acid ester resin (yield: 18%).

The resin composition, weight average molecular weight, and $T_\beta$ of the obtained fumaric acid ester resin were measured. The results thereof are shown together in Table 2. The obtained fumaric acid ester resin had a high $T_\beta$ and high weight average molecular weight.

[Chem. 33]

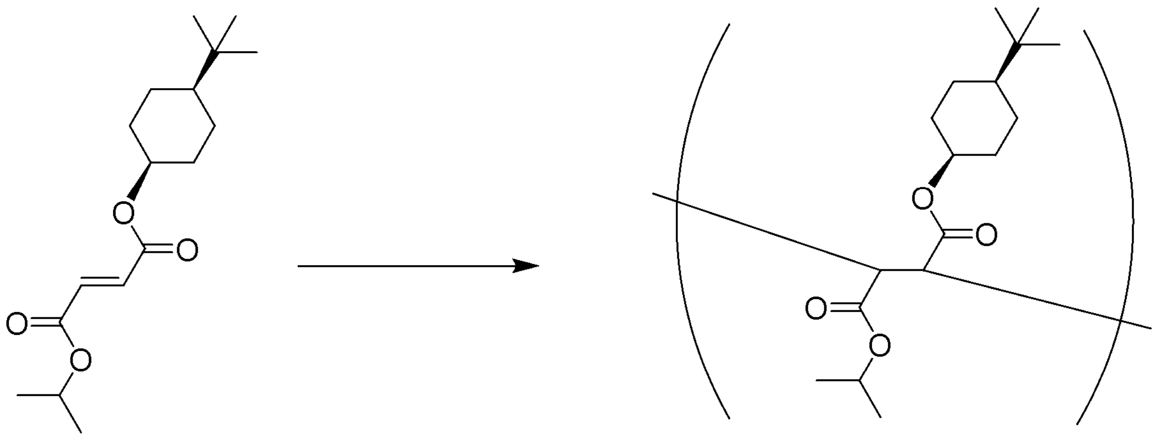

[Table 1]

| Reagent name | Manufacturer name | Trade name (content) |
|---|---|---|
| maleic anhydride | Nacalai Tesque, Inc. | - |
| ethanol | Nacalai Tesque, Inc. | - |
| isopropanol | FUJIFILM Wako Pure Chemical Industries | - |
| cyclohexanol | FUJIFILM Wako Pure Chemical Industries | - |
| 4-tert-butylcyclohexanol (cis/trans mixture) | Tokyo Chemical Industry | - |
| 4-tert-butylcyclohexanol (cis isomer) | Tokyo Chemical Industry | - |
| N,N'-dicyclohexylcarbodiimide | Nacalai Tesque, Inc. | - |
| 4-dimethylaminopyridine (DMAP) | FUJIFILM Wako Pure Chemical Industries, Nacalai Tesque, Inc. | - |
| piperidine | Nacalai Tesque, Inc. | - |
| ethylenediamine | Nacalai Tesque, Inc. | - |
| dicyclomethane | FUJIFILM Wako Pure Chemical Industries | - |
| cyclohexane | FUJIFILM Wako Pure Chemical Industries | - |
| ethyl acetate | Nacalai Tesque, Inc. | - |
| chloroform | Nacalai Tesque, Inc. | - |
| tetrahydrofuran | Nacalai Tesque, Inc. | - |
| toluene | Nacalai Tesque, Inc. | - |
| tert-butyl perhydroxyoxide | Tokyo Chemical Industry | - |
| tert-butyl peroxypivalate | NOF Corp. | Perbutyl PV (70%) |
| tert-butylperoxy-2-ethylhexanoate | NOF Corp. | Perbutyl O (97%) |
| diisopropyl fumarate (DiPF) | Tokyo Chemical Industry | - |
| hexane | Nacalai Tesque, Inc. | - |
| diethylether | FUJIFILM Wako Pure Chemical Industries | - |
| diethyl fumarate(DEF) | Tokyo Chemical Industry | - |

[Table 2]

| | Polymerization conditions | | | | Fumaric acid ester resin | | |
|---|---|---|---|---|---|---|---|
| | Monomer 1 | | Monomer 2 | Polymerization temperature | Resin composition (M1/M2) | Weight average molecular weight (Mw) | Tβ |
| | M1 | Trans ratio | M2 | (°C) | (mol%/mol%) | ($\times 10^4$) | (°C) |
| Example 4 | BCEF | - | - | 60 | 100/0 | 9.6 | 147 |
| Example 5 | BCiPF | 92.7% | - | 45 | 100/0 | 56.6 | 167 |
| Example 6 | BCCHF | - | - | 60 | 100/0 | 20.1 | 180 |
| Example 7 | BCEF | - | DiPF | 60 | 93/7 | 9.3 | 145 |
| Example 8 | BCiPF | 92.7% | DiPF | 45 | 84/16 | 48.5 | 169 |
| Example 9 | BCiPF | 92.7% | DiPF | 45 | 66/34 | 46.9 | 155 |
| Example 15 | BCiPF | 90.8% | DiPF | 45 | 42/58 | 48.0 | 138 |
| Example 16 | BCiPF | 90.8% | - | 50 | 100/0 | 49.0 | 173 |
| Example 17 | BCiPF | 90.3% | DEF | 50 | 84/16 | 9.7 | 142 |

(continued)

| | Polymerization conditions | | | | Fumaric acid ester resin | | |
|---|---|---|---|---|---|---|---|
| | Monomer 1 | | Monomer 2 | Polymerization temperature | Resin composition (M1/M2) | Weight average molecular weight (Mw) | Tβ |
| | M1 | Trans ratio | M2 | (°C) | (mol%/mol%) | ($\times 10^4$) | (°C) |
| Example 18 | BCiPF | 0.4% | - | 50 | 100/0 | 5.9 | 181 |
| Comparative Example 1 | DiPF | - | - | 50 | 100/0 | 26.2 | 70 |
| Comparative Example 2 | CHiPF | - | - | 45 | 100/0 | 26.1 | 117 |
| Comparative Example 3 | DBCF | - | - | 172 | 100/0 | 1.6 | 163 |

[Table 3]

| | Resin | | Film | |
|---|---|---|---|---|
| | Type | Tβ | Linear expansion coefficient α | Rth |
| | - | (°C) | (ppm/°C) | (nm) |
| Example 10 | Example 5 | 167 | 75 | -91 |
| Example 11 | Example 9 | 155 | 85 | -78 |
| Comparative Example 4 | Comparative Example 1 | 70 | 125 | -81 |
| Comparative Example 5 | Comparative Example 2 | 117 | 97 | -83 |

**Claims**

**1.** A fumaric acid ester monomer represented by formula (1) below, wherein

[Chem. 1]

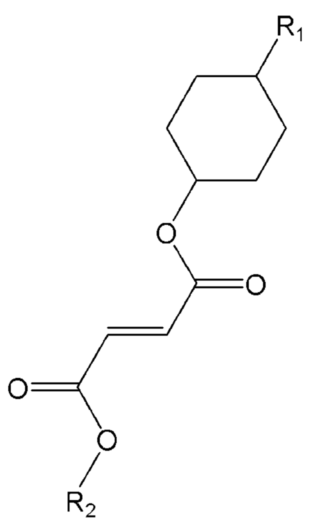

(1)

$R_1$ represents a linear alkyl group having 1 to 4 carbon atoms or a branched alkyl group having 3 to 5 carbon

atoms, and

$R_2$ represents a linear alkyl group having 1 to 4 carbon atoms, a branched alkyl group having 3 to 12 carbon atoms or a cyclic alkyl group having 3 to 6 carbon atoms.

**2.** The fumaric acid ester monomer according to claim 1, wherein $R_1$ is a methyl group, an ethyl group, a propyl group, an isopropyl group, a butyl group, a tert-butyl group, an isobutyl group, a sec-butyl group, a sec-pentyl group or a tert-pentyl group.

**3.** The fumaric acid ester monomer according to claim 1 or 2, wherein $R_2$ is a methyl group, an ethyl group, a propyl group, an isopropyl group, a butyl group, an isobutyl group, a sec-butyl group, a sec-pentyl group, a tert-pentyl group, a sec-hexyl group, a tert-hexyl group, a cyclopropyl group, a cyclopentyl group or a cyclohexyl group.

**4.** A fumaric acid ester monomer according to claim 1 or 2, wherein the fumaric acid ester monomer has a melting point of 80°C or less.

**5.** A resin comprising a residue unit represented by structural formula (2) below, wherein

[Chem. 2]

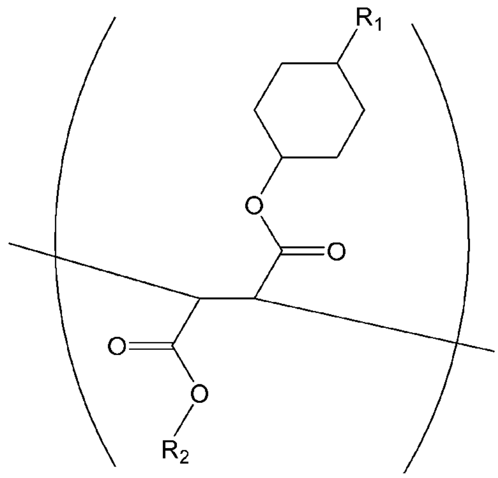

(2)

$R_1$ represents a linear alkyl group having 1 to 4 carbon atoms or a branched alkyl group having 3 to 5 carbon atoms, and

$R_2$ represents a linear alkyl group having 1 to 4 carbon atoms, a branched alkyl group having 3 to 12 carbon atoms or a cyclic alkyl group having 3 to 6 carbon atoms.

**6.** The resin according to claim 5, further comprising a residue unit represented by structural formula (3) below, wherein

[Chem. 3]

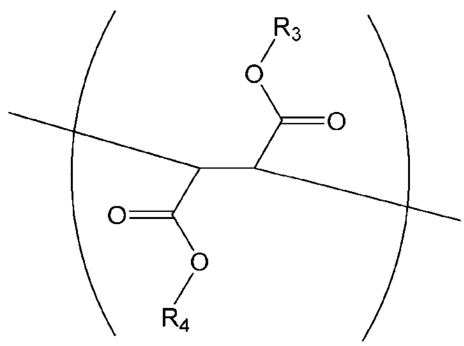

(3)

$R_3$ and $R_4$ each independently represent a linear alkyl group having 1 to 12 carbon atoms, a branched alkyl group having 3 to 12 carbon atoms or a cyclic alkyl group having 3 to 6 carbon atoms.

**7.** The resin according to claim 5 or 6, wherein a weight average molecular weight (Mw) of a standard polystyrene equivalent obtained from an elution curve measured by gel permeation chromatography (GPC) is 50,000 or more.

**8.** The resin according to claim 5 or 6, wherein a β relaxation temperature ($T_\beta$) during second scan temperature rise (temperature rise rate = 10°C /min) measured by differential scanning calorimetry (DSC) is 130°C or higher.

**9.** A film comprising the resin according to claim 5 or 6.

**10.** The film according to claim 9, wherein an out-of-plane retardation (Rth) measured at a wavelength of 589 nm represented by formula (a) below is -700 to 0 nm,

$$Rth = ((nx+ny)/2-nz) \times d \quad (a)$$

wherein nx represents a refractive index in a fast axis direction (direction with smallest refractive index) in a film plane, ny represents a refractive index in a slow axis direction in a film plane, nz represents a refractive index in a perpendicular direction outside a film plane, and d represents a film thickness.

**11.** The film according to claim 9, wherein a linear expansion coefficient α represented by formula (b) below is 95 ppm/°C or less,

$$\alpha=\Delta l/(\Delta T \times l) \quad (b)$$

wherein Δl represents a film length change amount during temperature change, ΔT represents a film temperature change amount, and l represents a film length prior to temperature change.

**INTERNATIONAL SEARCH REPORT**

International application No.
**PCT/JP2024/016031**

**A. CLASSIFICATION OF SUBJECT MATTER**

***C08F 22/14***(2006.01)i; ***C08J 5/18***(2006.01)i; *G09F 9/00*(2006.01)n
FI: C08F22/14; C08J5/18 CER; G09F9/00 313

According to International Patent Classification (IPC) or to both national classification and IPC

**B. FIELDS SEARCHED**

Minimum documentation searched (classification system followed by classification symbols)

C08F22/14

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | JP 3-125115 A (TOME SANGYO KK) 28 May 1991 (1991-05-28) claims, p. 3, upper right column, line 13 to lower right column, line 11, p. 5, upper right column, lines 3-9 | 1-7, 9 |
| A | | 8, 10-11 |
| A | JP 2009-37007 A (NOF CORPORATON) 19 February 2009 (2009-02-19) | 1-11 |
| A | JP 2000-159970 A (NOF CORP.) 13 June 2000 (2000-06-13) | 1-11 |
| A | JP 2013-28741 A (TOSOH CORP.) 07 February 2013 (2013-02-07) | 1-11 |
| A | JP 9-208627 A (TDK CORP.) 12 August 1997 (1997-08-12) | 1-11 |
| A | JP 5-132522 A (NOF CORP.) 28 May 1993 (1993-05-28) | 1-11 |
| A | JP 2004-168915 A (NOF CORP.) 17 June 2004 (2004-06-17) | 1-11 |

☐ Further documents are listed in the continuation of Box C.   ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 July 2024** | **16 July 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2024/016031**

| Patent document cited in search report | Publication date (day/month/year) | Patent family member(s) | Publication date (day/month/year) |
|---|---|---|---|
| JP 3-125115 A | 28 May 1991 | (Family: none) | |
| JP 2009-37007 A | 19 February 2009 | (Family: none) | |
| JP 2000-159970 A | 13 June 2000 | EP 1006130 A1<br>KR 10-2000-0035775 A | |
| JP 2013-28741 A | 07 February 2013 | US 2014/0153096 A1<br>WO 2013/018651 A1<br>EP 2738205 A1<br>TW 201321439 A<br>CN 103717660 A<br>KR 10-2014-0047684 A | |
| JP 9-208627 A | 12 August 1997 | US 5914283 A<br>EP 788118 A1<br>KR 10-1997-0063283 A | |
| JP 5-132522 A | 28 May 1993 | (Family: none) | |
| JP 2004-168915 A | 17 June 2004 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2008064817 A
- JP 2011107281 A